# FASCICULE DE BREVET EUROPEEN

(11) **EP 2 310 517 B1**
(45) Date de publication et mention de la délivrance du brevet: **16.03.2016**
(21) Numéro de dépôt: 09784257.9
(22) Date de dépôt: 08.07.2009
(51) Int. Cl.: C12P 7/04, C12N 9/04, C12N 1/19, C12N 15/29, C12N 15/81, C12P 7/64

(54) **NOUVELLES SOUCHES DE LEVURE MUTANTES CAPABLES D'ACCUMULER UNE GRANDE QUANTITÉ DE LIPIDES**
NEUE HEFESTAMMMUTANTEN MIT FÄHIGKEIT ZUR ANHÄUFUNG GROSSER LIPIDMENGEN
NEW MUTANT YEAST STRAINS CAPABLE OF ACCUMULATING A LARGE QUANTITY OF LIPIDS

(30) Priorité: 11.07.2008 FR 0854786
(43) Date de publication de la demande: 20.04.2011
(73) Titulaire: Institut National De La Recherche Agronomique (INRA), 75007 Paris (FR); Centre National de la Recherche Scientifique (CNRS), 75016 Paris (FR)
(72) Inventeur: NICAUD, Jean-Marc, F-78190 Trappes (FR); CHARDOT, Thierry, F-78530 Buc (FR); BEOPOULOS, Athanasios, F-75006 Paris (FR)
(74) Mandataire: Santarelli
(86) Numéro de dépôt international: PCT/FR2009/000848
(87) Numéro de publication internationale: WO 2010/004141

(56) Documents cités:
- THEVENIEAU ET AL: "Characterization of Yarrowia lipolytica mutants affected in hydrophobic substrate utilization" FUNGAL GENETICS AND BIOLOGY, SAN DIEGO, CA, US, vol. 44, no. 6, 6 mai 2007 (2007-05-06), pages 531-542, XP022065224 ISSN: 1087-1845
- WANG H J ET AL: "Evaluation of acyl coenzyme A oxidase (Aox) isozyme function in the n-alkane-assimilating yeast Yarrowia lipolytica" JOURNAL OF BACTERIOLOGY, AMERICAN SOCIETY FOR MICROBIOLOGY, US, vol. 181, no. 17, 1 septembre 1999 (1999-09-01), pages 5140-5148, XP002336152 ISSN: 0021-9193
- MLICKOVA K ET AL: "Acyl-CoA oxidase, a key step for lipid accumulation in the yeast Yarrowia lipolytica" JOURNAL OF MOLECULAR CATALYSIS B: ENZYMATIC 20040504 ELSEVIER NL, vol. 28, no. 2-3, 4 mai 2004 (2004-05-04), pages 81-85, XP002515888
- MLICKOVA K ET AL: "Lipid accumulation, lipid body formation, and acyl coenzyme A oxidases of the yeast Yarrowia lipolytica" APPLIED AND ENVIRONMENTAL MICROBIOLOGY JULY 2004 AMERICAN SOCIETY FOR MICROBIOLOGY US, vol. 70, no. 7, juillet 2004 (2004-07), pages 3918-3924, XP002515889
- RONNOW B ET AL: "GUT2, A GENE FOR MITOCHONDRIAL GLYSEROL 3-PHOSPHATE DEHYDROGENASE OF SACCHARMYCES CEREVISIAE", YEAST, JOHN WILEY & SONS LTD, GB, vol. 9, no. 10, 1 October 1993 (1993-10-01), pages 1121-1130, XP000563881, ISSN: 0749-503X, DOI: 10.1002/YEA.320091013

### SEQUENCE LISTING

<110> INRA
<120> Nouvelles souches de levure mutantes capables d'accumuler une grande quantité de lipides.
<130> BIF117612
<160> 23
<170> PatentIn version 3.3
<210> 1
   <211> 4140
   <212> DNA
   <213> Yarrowia lipolytica
<220>
   <221> Fragment P-GUT2-T
   <222> (1)..(4140)
   <223> ADN contenant le fragment P et le fragfₘent T encadrant le gène GUT 2
<220>
   <221> Ver1
   <222> (1)..(2-0)
   <223> Amorce ver 1 de vérification de l'invalidation du gène GUT2
<220>
   <221> G3P-P1
   <222> (17)..(36)
   <223> Amorce P1 de synthèse du fragment P du gène GUT 2
<220>
   <221> G3P-P2
   <222> (1114)..(1135)
   <223> Amorce P2 de synthèse du fragment P du gène GUT 2
<220>
   <221> Gène GUT 2
   <222> (1151)..(2989)
   <223> séquence codante du gène GUT 2
<220>
   <221> G3P-T1
   <222> (3067)..(3085)
   <223> Amorce T1 de synthèse du fragment T du gène GUT 2
<220>
   <221> G3P-T2
   <222> (3863)..(3883)
   <223> Amorce T2 de synthèse du fragment T du gène GUT 2
<220>
   <221> Ver2
   <222> (44116)..(4140)
   <223> Amorce ver 2 de vérification de l'invalidation du gène GUT2
<400> 1
   gaatgacggg ggcaacgcag atccactgtc aagccgagtc tgtccgccac gcatcatgtg 60
   atagaggcta gggagtcatg tgaatatgtc acacctggat gacctctaca tacaagagcg 120
   gtttgttttc tatggcatgt tgttgacgca tgctgccaac ggctattcaa cggtgacaac 180
   ggatgatgct gtcacatgac gccatttttt atgttgtatc caacagcacg gtactaaaac 240
   aggccatttg taaaggcctc actcagctca cacacgctca acggtcacga taaggtcgca 300
   ctagaggcgt tagttggttt caagaatagt ggttattggt cttgggatac gggttggaca 360
   atatacaaat gggctcgcgt acacttatac agtcctacca ttctgtcgcc ctctgattct 420
   ccgccacatc agccacgccg caacgtctcc tcctcatccc cctcctgctc ttccactcgc 480
   aaaacgtcca aactcaattg tgtcaaaatt ggaggttctc ttcgtttgag cctaccattt 540
   tcaatttttt agttgcgaca gcggcccggt cagaggttca caacaaggtc tagagacact 600
   ttgtcatggg gccgagaagg accataaaaa ccaaacgatg gtcacgtcag gtcaattact 660
   gaccagtctc acatccgacc cctcgcgtgc tcgaccggag gatttctctg cactcgtcct 720
   tgcatacctc ggctagcggg atttattcac caatcacaca gccgagagtt tttccggacc 780
   cttcatccaa cagcttagag ttgcatgagt cagtagcaac gtagactttg agcctttgtg 840
   acagatgtcc aagtgcagca cgttgtagga aaataaggtg aaggattggc caatgtgaac 900
   agaggcgaca agagtccgtc tggagggctt gttgtagtca attgcccgcg caattgattg 960
   acctcatcgt ttctgccgga acccccccac aagcccggat aaatagacac gccccacaag 1020
   ccgttcgtct ggtctgctca cagcacactt ccatttaaaa ttcaaacaaa gcgcaccacc 1080
   gcaaagcata cttaacccac tcaatgtaga cgtcgcggaa cttctctttc ctacccacca 1140
   ccccaaacaa atgttcagaa ccattcgaaa acccgcgtgg gctgctgccg ccgccgtggc 1200
   agccgctggc gctggagccg tcgccctgtc tgtgcctgcc caggcccagg aggagctcca 1260
   caagaagcac aaattcacag tgccccccgt ggccgccgag cccccctctc gagccgccca 1320
   gctcgagaag atgaagaccg aggagtttga tctcgtcgtt gttggtggag gagctaccgg 1380
   atccggtatc gccctcgacg ctgtcacacg aggcctcaag gttgctctgg tcgagcgaga 1440
   cgatttctcc tgcggaacct cgtcccgatc caccaagctc atccacggag gtgtccgata 1500
   cctcgagaag gctgtgtgga acctcgacta caaccagtac gagctggtca aggaggccct 1560
   gcacgagcga aaggtcttcc tcgacattgc tccccacctc acctttgctc tgcccatcat 1620
   gatccccgtc tacacctggt ggcagcttcc ctacttctgg atgggtgtca agtgctacga 1680
   tctgcttgcc ggccgacaga acctcgagtc ctcttacatg ctctcccgat cccgtgctct 1740
   cgatgccttc cccatgcttt ccgatgacaa gctcaagggc gccattgtct actatgatgg 1800
   ctcccagaac gactctcgaa tgaacgtttc tcttattatg actgctgttg agaagggtgc 1860
   caccatcctg aaccattgcg aggtcaccga gctcaccaag ggcgccaatg gccagctcaa 1920
   cggtgttgtt gccaaggata ctgacggaaa cgctggatcc ttcaacatca aggccaagtg 1980
   tgtcgttaat gctactggac ccttcactga ctctctgcga cagatggacg acaagaacac 2040
   caaggagatc tgtgctcctt cctccggtgt tcacatcatt ctccccggtt actactcccc 2100
   caagaagatg ggactccttg accccgctac ttctgacggc cgagttatct tcttcctccc 2160
   ctggcaggga aacacccttg ccggtactac tgaccagcct accaagatca ctgctaaccc 2220
   tatcccctcc gaggaggaca ttgacttcat tctcaacgag gtccgacact acgttgaggg 2280
   caaggttgat gtgcgacgag aggacgttct ggccgcctgg tccggaatcc gaccccttgt 2340
   ccgggacccc cacgccaaga acaccgagtc tcttgtccga aaccatctca tcacctactc 2400
   cgagtctggt cttgtcacca ttgctggcgg aaagtggacc acttaccgac agatggctga 2460
   ggagactgtc gatgcctgca ttgccaagtt cggtctcaag cctgaaatct ccgccaaggc 2520
   cgtcacccga gacgtcaagc tcatcggtgc taaggactgg actcctctca cttacattga 2580
   tctgatccag caggaggacc ttgaccccga ggttgctaag cacctttctg agaactacgg 2640
   atctcgagct ttcaccgttg cttctcttgc tgagatgccc acccccgaac ccggtgtgat 2700
   cccccagtct actctcacaa agggtaagcg aatcctgtac ccctacccct acctcgatgc 2760
   cgagtgcaag tactctatga agtacgagta tgccaccacc gccatcgact tccttgctcg 2820
   acgaactcgt cttgctttcc ttaacgccgc tgccgcctac gaggctctcc ctgaggtcat 2880
   tgagatcatg gccaaggagc tccagtggga cgaggctcga aaggagcagg aattcaacac 2940
   cggtgtcgag tacctctact ccatgggcct tacccccaag gacaaataac tgtatagtaa 3000
   aagcgtatag ccaataagat aatcacttga atgaaggagc agcaactcgt atgtttagca 3060
   cttcaacgga ctatttcccc gcagcaaaga gactattgct gagttgttga gtatctgctt 3120
   tacaataatg gggtatggac acacaaggag gggtcttagt gagaagttag ataggtctag 3180
   catacatgag atcaatgtgg tcttacctat atcgtttgtt atcatttatc ttggtttgaa 3240
   ttgataacac gagttgttca ttgaagtgat ggcaccgggt ctcacacgca acagttggcg 3300
   aacaggtcgt attgttcctt agatacgacg ctcttttgga catgatggga agtgaaacta 3360
   caattacagt agctacatag cttggctaac tagaccgctt acagaaccag tagtcgtcac 3420
   aagaccacca cgaacaaagt ccaactaccc cactcccacc actcgtattt acttaccgca 3480
   gatcacacgc ttcggtgtat ctccgtgggg catcgtgggg cattgttcta agttttccgt 3540
   atggtgcaca gtcggtacgt gctttgacta accagtagaa gttaggctac tgtagtggag 3600
   attgagcaat gaaacgatga caggaagacc ccaaaatgcg accacctcaa ctatacacgg 3660
   cttgttgcta ttgccgcctt gcactccaca cagcaaacat gcacacgata tgcactcaag 3720
   tcttaaccga atgaaggtaa aagtagcaac caacaagcga gagttactgt atacttacaa 3780
   gttatacgac agtctcactt atcaccaatt ggcaacttga ccgcacagac aaacacccta 3840
   caatgacctt cctcaatgtg ctctactacg tgctgctggc tgccatcatg atcggcaccg 3900
   gctacttcta ctacctgtgg ttcactgaga ccaacgacca aaccgagaag atcatcagag 3960
   ctgcgcttgg agtctttgat atcgccatct ggtacattct aggtatctcc acctccttta 4020
   agatcctcac ccagatgatt ctcgcctgtt tccttgtggt tctggctggt cttaagatct 4080
   acatcaaccc tcgagttgga ggggctcttc tggccggcag tctgctattt gtggctgctg 4140
<210> 2
   <211> 20
   <212> DNA
   <213> unidentified
<220>
   <221> G3P-P1
   <222> (1)..(20)
   <223> Amorce pour la synthèse du fragment P du gène GUT2
<400> 2
   gcagatccac tgtcaagccg 20
<210> 3
   <211> 44
   <212> DNA
   <213> unidentified
<220>
   <221> G3P-P2
   <222> (1)..(44)
   <223> Amorce pour la synthèse du fragment P du gène GUT2
<220>
   <221> G3P-P2
   <222> (1)..(22)
   <223> Site I-SceI dans l'amorce pour la synthèse du fragment P du gène GUT2
<400> 3
   gctagggata acagggtaat gcggtaggaa agagaagttc cgcg 44
<210> 4
   <211> 41
   <212> DNA
   <213> unidentified
<220>
   <221> G3P-T1
   <222> (1)..(41)
   <223> Amorce pour la synthèse du fragment T du gène GUT2
<220>
   <221> G3P-T1
   <222> (1)..(22)
   <223> Site I-SceI dans l'amorce pour la synthèse du fragment T du gène GUT2
<400> 4
   gcattaccct gttatcccta gccggactat ttccccgcag c 41
<210> 5
   <211> 21
   <212> DNA
   <213> unidentified
<220>
   <221> G3P-T2
   <222> (1)..(21)
   <223> Amorce pour la synthèse du fragment T du gène GUT2
<400> 5
   gcagccagca gcacgtagta g 21
<210> 6
   <211> 20
   <212> DNA
   <213> unidentified
<220>
   <221> G3P-ver1
   <222> (1)..(20)
   <223> Amorce pour la vérification de l'invalidation du gène GUT2
<400> 6
   gaatgacggg ggcaacgcag 20
<210> 7
   <211> 25
   <212> DNA
   <213> unidentified
<220>
   <221> G3P-ver2
   <222> (1)..(25)
   <223> Amorce pour la vérification de l'invalidation du gène GUT2
<400> 7
   cagcagccac aaatagcaga ctgcc 25
<210> 8
   <211> 34
   <212> DNA
   <213> unidentified
<220>
   <221> LEU2-P1
   <222> (1)..(34)
   <223> Amorce pour la synthèse du fragment P du gène LEU2
<220>
   <221> LEU2-P1
   <222> (3)..(8)
   <223> Site XbaI dans l'amorce pour la synthèse du fragment P du gène LEU2
<400> 8
   aatctagatg gtcacagtgg aatcatgttc gtgg 34
<210> 9
   <211> 45
   <212> DNA
   <213> unidentified
<220>
   <221> LEU2-P2
   <222> (1)..(45)
   <223> Amorce pour la synthèse du fragment P du gène LEU2
<220>
   <221> LEU2-P2
   <222> (1)..(20)
   <223> Site I-SceI dans l'amorce pour la synthèse du fragment P du gène LEU2
<400> 9
   cattaccctg ttatccctag gttccattgt ggatgtgtgt ggttg 45
<210> 10
   <211> 39
   <212> DNA
   <213> unidentified
<220>
   <221> LEU2-T1
   <222> (1)..(39)
   <223> Amorce pour la synthèse du fragment T du gène LEU2
<220>
   <221> LEU2-T1
   <222> (1)..(20)
   <223> Site I-SceI dans l'amorce pour la synthèse du fragment T du gène LEU2
<400> 10
   ctagggataa cagggtaatg ctctgggtct gctgccctc 39
<210> 11
   <211> 41
   <212> DNA
   <213> unidentified
<220>
   <221> LEU2-T2
   <222> (1)..(41)
   <223> Amorce pour la synthèse du fragment T du gène LEU2
<220>
   <221> LEU2-T2
   <222> (4)..(10)
   <223> Site Hind III dans l'amorce pour la synthèse du fragment T du gène LEU2
<400> 11
   agtaagctta gatctgttcg gaaatcaacg gatgctcaac c 41
<210> 12
   <211> 24
   <212> DNA
   <213> unidentified
<220>
   <221> POX1-P1
   <222> (1)..(24)
   <223> Amorce pour la synthèse du fragment P du gène POX1
<400> 12
   catggagtgg atcgctcgag gacg 24
<210> 13
   <211> 42
   <212> DNA
   <213> unidentified
<220>
   <221> POX1-P2
   <222> (1)..(42)
   <223> Amorce pour la synthèse du fragₘent P du gène POX1
<220>
   <221> POX1-P2
   <222> (1)..(22)
   <223> Site I-SceI dans m'amorce pour la synthèse du fragment P du gène POX1
<400> 13
   gcattaccct gttatcccta gccaggagga tcggtgaatg tg 42
<210> 14
   <211> 45
   <212> DNA
   <213> unidentified
<220>
   <221> POX1-T1
   <222> (1)..(45)
   <223> Amorce pour la synthèse du fragment T du gène POX1
<220>
   <221> POX1-T1
   <222> (1)..(22)
   <223> Site I-SceI dans l'amorce pour la synthèse du fragₘent T du gène POX1
<400> 14
   gctagggata acagggtaat gccttgttcc gagaagagga ggacg 45
<210> 15
   <211> 19
   <212> DNA
   <213> unidentified.
<220>
   <221> POX1-T2
   <222> (1)..(19)
   <223> Amorce pour la synthèse du fragment T du gène POX1
<400> 15
   cggcagtggc tcaccaagc 19
<210> 16
   <211> 19
   <212> DNA
   <213> unidentified
<220>
   <221> POX1-ver1
   <222> (1)..(19)
   <223> Amorce pour la vérification de l'invalidation du gène POX1
<400> 16
   atccagacct ccaggcggg 19
<210> 17
   <211> 23
   <212> DNA
   <213> unidentified
<220>
   <221> POX1-ver2
   <222> (1)..(23)
   <223> Amorce pour vérification de l'invalidation du gène POX1
<400> 17
   gctgcgtctc aatctggcga atg 23
<210> 18
   <211> 28
   <212> DNA
   <213> unidentified
<220>
   <221> POX6-P1
   <222> (1)..(28)
   <223> Amorce pour la synthèse du fragment P du gène POX6
<400> 18
   ccaagctcta agatcatggg gatccaag 28
<210> 19
   <211> 43
   <212> DNA
   <213> unidentified
<220>
   <221> POX6-P2
   <222> (1)..(43)
   <223> Amorce pour la synthèse du fragₘent P du gène POX6
<220>
   <221> POX6-P2
   <222> (1)..(22)
   <223> Site i-SceI dans l'amorce pour la synthèse du fragment P du gène POX6
<400> 19
   gcattaccct gttatcccta gcgttgaggg actgttgaga gag 43
<210> 20
   <211> 47
   <212> DNA
   <213> unidentified
<220>
   <221> POX6-T1
   <222> (1)..(47)
   <223> Amorce pour la synthèse du fragment T du gène POX6
<220>
   <221> POX6-T1
   <222> (1)..(22)
   <223> Site I-SceI dans l'amorce pour la synthèse du fragment T du gène POX6
<400> 20
   gctagggata acagggtaat gcgatgagga aatttgctct cttgagg 47
<210> 21
   <211> 28
   <212> DNA
   <213> unidentified
<220>
   <221> POX6-T2
   <222> (1) .. (28)
   <223> Amorce pour la synthèse du fragment T du gène POX6
<400> 21
   atctcgagat tggtcccctc aaacacac 28
<210> 22
   <211> 21
   <212> DNA
   <213> unidentified
<220>
   <221> POX6-ver1
   <222> (1)..(21)
   <223> Amorce pour la vérification de l'invalidation du gène POX6
<400> 22
   gctcaagaag gtagctgagt c 21
<210> 23
   <211> 23
   <212> DNA
   <213> unidentified
<220>
   <221> POX6ver2
   <222> (1)..(23)
   <223> Amorce pour la vérification de l'invalidation du gène POX6
<400> 23
   cattaagtgt cagatcagct cgc 23

## Description

L'engouement croissant pour les énergies de substitutions a entraîné un accroissement des recherches pour des sources d'énergie substitutives.

Plusieurs technologies telles que la fermentation à grande échelle, sont appliquées pour la production industrielle d'huile à partir de microorganismes en utilisant comme substrat des matières grasses ou du glycérol.

Parmi les applications industrielles potentielles de ces processus on distingue particulièrement l'accumulation dans des microorganismes de lipides enrichis en acides gras essentiels, lesdits lipides étant particulièrement destinés à être utilisés comme complément nutritionnel, ou pour la production de combustible sous la forme d'énergie renouvelable en alternative au pétrole.

Du fait de l'importance économique grandissante des sources d'énergie renouvelables, on constate un intérêt grandissant pour l'amélioration de la composition et de la teneur en huile des microorganismes, particulièrement des levures.

A cet égard la levure oléagineuse *Yarrowia lipolytica*, est une des levures "non conventionnelles" les plus largement étudiées, en raison de sa capacité d'accumuler une grande quantité de lipides (environ 40% de son poids sec).

Les levures, particulièrement *Yarrowia lipolytica*, sont capables d'utiliser efficacement des substrats hydrophobes, par exemple des alcanes, des acides gras et des huiles, comme seule source de carbone.

Les chaînes aliphatiques ingérées peuvent être utilisées pour la production d'énergie ou accumulées sous des formes inchangées ou modifiées.

Les molécules de stockage telles que les triglycérides (TG) et/ou les esters de stérols, (sterylesters ; SE), incapables de s'intégrer dans les bicouches de phospholipides, se groupent pour former le noyau hydrophobe desdites corps lipidiques (lipid bodies ; LB).

Lesdits corps lipidiques ont longtemps été uniquement considérées comme un stockage de lipides neutres pouvant être mobilisés en période de privation.

Toutefois, l'image desdites corps lipidiques comme simple compartiment de stockage a dû être révisée depuis que de nombreuses protéines desdites corps lipidiques ont été identifiées comme des enzymes impliquées dans le métabolisme lipidique, en particulier dans la synthèse et/ou la dégradation des triglycérides.

Dans les levures, la synthèse des triglycérides suit la voie Kennedy. Les acides gras libres sont activés pour le coenzyme A (CoA) et utilisés pour l'acylation du glycérol, pivot de la synthèse des triglycérides.

Dans la première étape d'assemblage de triglycérides, le glycérol-3-phosphate (G-3-P) est acylé par l'acyltransférase spécifique du glycérol-3-phosphate (glycerol-3-phosphate acyltransférase ou SCT1) pour donner de l'acide lysophospatidique, qui est ensuite acylé par l'acyltransférase spécifique de l'acide lysophosphatidique (Phosphatidic acid acyltransferase ou SLC1) pour donner de l'acide phosphatidique (PA). Celui-ci est ensuite déphosphorylé par une phosphohydrolase spécifique de l'acide phosphatidique (phosphatidic acid phosphohydrolase (PAP)) pour libérer du diacylglycérol (DAG).

Dans la dernière étape le diacylglycérol est acylé soit par une diacylglycérol acyltransférase soit par une phospholipide diacylglycérol acyltransférase pour produire des Triglycérides

Le tableau 1 suivant décrit les gènes impliqués dans le métabolisme des acides gras chez les levures, particulièrement chez *Yarrowia lipolytica,* (YL).

**Tableau 1**

| Gène | Nom | N°EC | Fonction |
|---|---|---|---|
| GUT1 | YALI0F00484g | EC 2.7.1.30 | Glycerol kinase |
| GPD1 | YALI0B02948g | EC 1.1.1.18 | Glycerol-3-phosphate dehydrogenase (NAD(+)) |
| GUT2 | YALI0B13970g | EC 1.1.99.5 | Glycerol-3-phosphate dehydrogenase |
| SCT1 | YALI0C00209g | EC 2.3.1.15 | Glycerol-3-phosphate acyl transferase |
| SLC1 | YALI0E18964g | EC 2.3.1.51 | 1-acyl-sn-glycerol-3-phosphate acyltransferase |
| DGA1 | YALI0E32769g | EC 2.3.1.20 | Diacylglycerol acyltransferase |
| LRO1 | YALI0E16797g | EC 2.3.1.158 | Phospholipid:diacylglycerol acyltransferase |
| TGL3 | YALI0D17534g | EC 3.1.1.3 | Triacylglycerol lipase |
| TGL4 | YALI0F10010g | EC 3.1.1.3 | Triacylglycerol lipase |
| ARE1 | YALI0F06578g | EC 2.3.1.26 | Acyl-CoA:sterol acyltransferase |
| TGL1 | YALI0E32035g | EC 3.1.1.13 | Cholesterol esterase |
| POX1 | YALI0E32835g | EC 6.2.1.3 | Acyl-coenzyme A oxidase |
| POX2 | YALI0F10857g | EC 6.2.1.3 | Acyl-coenzyme A oxidase |
| POX3 | YALI0D24750g | EC 6.2.1.3 | Acyl-coenzyme A oxidase |
| POX4 | YALI0E27654g | EC 6.2.1.3 | Acyl-coenzyme A oxidase |
| POX5 | YALI0C23859g | EC 6.2.1.3 | Acyl-coenzyme A oxidase |
| POX6 | YALI0E06567g | EC 6.2.1.3 | Acyl-coenzyme A oxidase |
| MFE1 | YALI0E15378g | EC 4.2.1.74 | Multifunctional beta-oxidation protein |
| POT1 | YALI0E18568g | EC 2.3.1.16 | Peroxisomal Oxoacyl Thiolase |

Dans les levures, la dégradation des acides gras se produit par une β-oxydation, un processus en plusieurs étapes nécessitant quatre activités enzymatiques différentes.

Chez les levures, les enzymes sont essentiellement localisées dans les peroxysomes, contrairement aux mammifères chez qui elles sont localisées dans les mitochondries et les peroxysomes.

La mobilisation des lipides accumulés se produit au cours des trois phases distinctes :
(i) au cours de la phase exponentielle, durant laquelle les composés lipidiques stockés sont utilisés pour la synthèse des lipides membranaires pour soutenir la croissance et la division cellulaire,
(ii) au cours de la phase stationnaire, lors de l'épuisement des nutriments, les acides gras libres sont libérés, plutôt lentement, à partir des triglycérides et soumis à β-oxydation peroxysomale ;
(iii) lorsque les cellules sortes de conditions de privation, par exemple en sortie de phase stationnaire et entrée en cycle de croissance végétative avec supplémentation en carbone, les dépôts lipidiques sont très rapidement dégradés en acides gras libres.

Au cours de leurs recherches, les inventeurs se sont focalisés sur l'importance du glycérol-3-phosphate (G-3-P) dans la formation des triglycérides.

Le glycérol-3-phosphate, localisée dans les inclusions lipidiques, a déjà été soupçonnés de jouer un rôle crucial dans le métabolisme des triglycérides.

Il existe deux voies de synthèse pour le glycérol-3-phosphate.

Dans la première, le glycérol-3-phosphate est dérivé du glycérol par l'intermédiaire de la glycérol kinase codée par le gène GUT1.

Dans la deuxième voie, le glycérol-3-phosphate est directement synthétisée à partir de la dihydroxyacétone phosphate (DHAP) catalysée par la glycérol-3-phosphate déshydrogénase (codée par le gène GPD1). Cette dernière réaction est réversible et la formation de DHAP à partir de glycérol-3-phosphate est catalysée par un deuxième isoforme de la glycérol-3-phosphate déshydrogénase, dont le gène est dénommé GUT2.

On comprend que dans le présent texte, l'utilisation du terme "levure" sous entend que l'on considère, même si cela n'est pas précisé, les souches levures en général et préférentiellement les souches de levure *Yarrowia lipolytica.*

De manière surprenante et inattendue, les inventeurs ont pour la première fois montré qu'une extinction concomitante du gène GUT2 et des gènes responsables de la β-oxydation des lipides, comme par exemple les gènes POX, partiellement (POX2 à POX5) ou totalement (POX1 à POX6), ou encore le gène MFE1 ou le gène POT1, afin de bloquer la voie de la β-oxydation, permet d'augmenter encore l'accumulation de lipides dans les levures, particulièrement chez *Yarrowia lipolytica.* En effet chez les levures, particulièrement chez *Yarrowia lipolytica,* 6 gènes POX1, POX2, POX3, POX4, POX5 et POX6 codent 6 isoformes d'acyl-CoA oxydases impliquées, au moins partiellement au niveau de la β-oxydation. L'extinction, partielle ou totale, de l'expression de ces gènes codants ces isoenzymes conduit à l'accumulation par les levures, particulièrement chez *Yarrowia lipolytica,* d'acide dodécanedioïque, sans consommation des lipides accumulés.

C'est sur la base de ces découvertes que les inventeurs proposent une souche mutante de levure *Yarrowia lipolytica,* n'exprimant pas le gène GUT2 et les gènes responsables de la β-oxydation des lipides, particulièrement les gènes POX (1 à 6), ladite souche mutante étant capable d'accumuler une grande quantité de lipides.

Le document Thevenieau et al, (Fungal Genetics and Biology, san Diego, CA, US, vol. 44, n° 6, 6 mai 2007, pages 531-542) divulgue une souche de levure de *Yarrowia lipolytica* n'exprimant pas le gène GUT2. Aucune indication n'est donnée sur sa capacité à accumuler des lipides.

L'invalidation du gène GUT2 (YALI0B13970g) permet d'obtenir des souches mutantes de levures, particulièrement de levures *Yarrowia lipolytica,* capables de présenter une augmentation du niveau de lipide total accumulé et une augmentation de la vitesse d'accumulation de lipides au cours de la croissance sur glucose et/ou sur lipides. Ces mutants sont aptes à produire des acides gras n-2 par blocage partiel de la β-oxydation (un seul cycle de β-oxydation). Ainsi en partant d'un acide gras 18:1 (n-9), on accumule le produit d'un cycle de β-oxydation qui est le 16:1 (n-9), l'acide (Z)-7-hexadedecenoique.

De même, l'invalidation concomitante du gène GUT2 (YALI0B13970g) et des gènes responsables de la β-oxydation des lipides, particulièrement des gènes POX permet d'obtenir des mutants de levures, particulièrement de *Yarrowia lipolytica,* capables de présenter une augmentation du niveau de lipide total accumulé, une augmentation de la vitesse d'accumulation de lipide au cours de la croissance sur glucose ou sur lipide et une absence de consommation des lipides accumulés.

Selon un mode particulier, l'invention a pour objet une nouvelle souche de levure, particulièrement une souche *Yarrowia lipolytica,* mutante, n'exprimant ni le gène GUT2, ni au moins un gène responsable de la β-oxydation des lipides, ladite souche mutante étant capable d'accumuler des lipides.

Selon un autre mode particulier, l'invention a pour objet une nouvelle souche de levure, particulièrement une souche *Yarrowia lipolytica,* mutante, n'exprimant ni le gène GUT2, ni au moins un gène responsable de la β-oxydation des lipides choisi parmi les gènes les gènes POX, MFE1 ou POT1, avantageusement les gènes POX, partiellement (POX2 à POX5) ou totalement (POX1 à POX6), ladite souche mutante étant capable d'accumuler des lipides.

Préférentiellement selon l'invention ladite souche *Yarrowia lipolytica,* mutante, n'exprimant ni le gène GUT2, ni les gènes POX1, POX2, POX3, POX4, POX5 et POX6, ladite souche mutante étant capable d'accumuler des lipides. Préférentiellement selon l'invention ladite souche est une souche JMY1393, |delta|gut2|delta|pox1-6, déposée auprès de la Collection Nationale de Cultures de Microorganismes (CNCM, Institut Pasteur, 25, rue du Docteur Roux, F-75724 Paris Cedex 15) sous le n°CNCM I-4169, le 28 mai 2009.

L'invention a aussi pour objet un procédé d'obtention d'une souche de levure, particulièrement une souche *Yarrowia lipolytica,* mutante, ladite souche mutante étant capable d'accumuler des lipides.

L'art antérieur décrit différentes méthodes pouvant permettre l'obtention de souches de levure, particulièrement des souches *Yarrowia lipolytica,* n'exprimant pas un gène.

Par exemple, on citera la méthode appelée POP IN/ POP OUT qui a été utilisée chez les levures, particulièrement chez *Yarrowia lipolytica,* pour la délétion des gènes LEU2, URA3 et XPR2 comme il est décrit dans la revue de G. Barth et coll. : (Yarrowia lipolytica, in: Nonconventional Yeasts in Biotechnology A Handbook (Wolf, K., Ed.), Vol. 1, 1996, pp. 313-388. Springer-Verlag). Elle consiste à intégrer un vecteur comprenant un gène d'intérêt délété au locus considéré, puis à sélectionner l'excision dudit vecteur et d'identifier un clone qui par recombinaison a éliminé le gène sauvage et conservé le gène muté.

Préférentiellement selon l'invention on pourra utiliser une méthode conduisant à l'invalidation du gène d'intérêt.

Par invalidation d'un gène d'intérêt, on entend selon l'invention, toute méthode aboutissant à la non expression de la protéine native codée par ledit gène d'intérêt, par modification de l'enchaînement des nucléotides constituant ledit gène de telle sorte que quand bien même sa traduction serait effective, elle ne conduirait pas à l'expression de la protéine native codée par le gène d'intérêt sauvage.

Préférentiellement selon l'invention, on utilise une méthode conduisant à une extinction totale de l'expression du gène d'intérêt. Cela peut être réalisé par une délétion total du gène d'intérêt, par une délétion partielle du gène d'intérêt, par l'insertion d'un ou plusieurs nucléotides dans ledit gène d'intérêt, ladite méthode utilisée rendant le gène d'intérêt non fonctionnel (gène d'intérêt invalidé), à tout le moins codant une protéine ne possédant pas les propriétés de la dite protéine native. On obtient ainsi une souche de levure n'exprimant pas le gène d'intérêt que l'on nommera par la suite dans le présent texte "souche défective en gène d'intérêt".

On peut ainsi utiliser la méthode SEP (Maftahi M., et coll. ; Yeast 12 : 859-868) qui a été adaptée chez *Yarrowia lipolytica* pour l'invalidation successive des gènes POX (Wang H. J. et coll., 1999 ; J. Bacteriol., 181:5140-5148). Avantageusement selon l'invention on utilisera la méthode SEP/cre développé par Fickers et coll. (2003, J. Microbiol. Methods 55/3:727-737) et décrite dans la demande internationale WO2006/064131. C'est une méthode rapide et qui ne nécessite pas l'utilisation d'un marqueur permettant une contre sélection.

En résumé cette méthode consiste à
1) sélectionner un gène d'intérêt de levure, particulièrement un gène de *Yarrowia lipolytica,* dont on veut supprimer l'expression, soit par délétion du gène, à tout le moins par invalidation ;
2) construire une cassette d'invalidation par Réaction de Polymérisation en Chaîne (Polymerase Chain Reaction : PCR) ou par clonage, comprenant les séquences promotrices (P) et terminatrices (T) du gène d'intérêt, encadrant un gène codant un marqueur de sélection (gène de sélection), ledit gène de sélection étant lui-même encadré de part et d'autre de sa séquence par une (ou des) séquence(s) de recombinaison, lesdites séquences de recombinaison permettant une recombinaison entre elles conduisant à l'élimination dudit gène codant le marqueur de sélection ;
3) introduire ladite cassette d'invalidation obtenue en 2, dans une souche de levure ;
4) sélectionner une souche de levure défective en gène d'intérêt, souche ayant introduit par double recombinaison (double cross-over) le gène marqueur en lieu et place du gène d'intérêt, conduisant ainsi à un gène d'intérêt invalidé ;
5) vérifier l'invalidation dudit gène d'intérêt dans ladite souche de levure défective en gène d'intérêt sélectionnée à l'étape 4 ; et éventuellement
6) transformer ladite souche sélectionnée à l'étape 5 avec un vecteur permettant l'expression d'une recombinase afin d'obtenir l'élimination du gène exprimant le marqueur de sélection ;
7) isoler une souche de levure défective en gène d'intérêt, et ayant perdu le plasmide d'expression de la recombinase.

Avantageusement, à l'étape 2, le cadre de lecture du gène d'intérêt est remplacé, par insertion d'un gène marqueur lui-même encadré par des séquences répétées pouvant permettre une recombinaison qui pourra conduire à l'élimination du gène marqueur. Avantageusement la ou les séquence(s) de recombinaison, est (ou sont) une (ou des) séquence(s) IoxP ou une (ou des) séquence(s) IoxR ou (une ou des) séquence(s) dérivée(s) de ces séquences de recombinaison, ladite (lesdites) séquence(s) dérivées(s) ayant conservée(s) l'activité des séquences de recombinaison d'origine.

Préférentiellement à l'étape 2, le gène codant le marqueur de sélection pourra être encadré par des séquences de type IoxP qui, sous l'action de la recombinase cre se recombinent entre elles en donnant naissance à un plasmide comprenant la séquence du gène codant ledit marqueur de sélection.

L'étape 3 peut être réalisée par toute méthode de transformation des levures connues de l'art antérieur. Avantageusement pour ce qui concerne la culture des souches de levure, comme d'ailleurs pour toutes les techniques de biologie moléculaire utilisables selon l'invention, on se référera, aux manuels de référence en la matière à savoir le Sambrook (Sambrook, J., T. Maniatis and E.F. Fritsch, Molecular cloning : a laboratory manual. 2nd ed. 2nd ed. 1989, Cold Spring Harbor, New York: Cold Spring Harbor Laboratory Press) ou au Barth (Barth, G. and C. Gaillardin, Nonconventional Yeasts in Biotechnology, W. K., Editor. 1996, Springer-Verlag: Berlin, Heidelberg, New York. p. 313-388).

L'étape 4 de la méthode consiste en la sélection des souches de levures transformées exprimant le marqueur de sélection.

L'étape 5 peut être réalisée selon toute méthode connue et en particulier par PCR selon la technique de Gussow et coll. (Nucleic Acids Res. 17, 1989, 4000), puis confirmée par hybridation Southern blot.

L'étape 6 peut être réalisée comme l'étape 3. Avantageusement on utilisera un plasmide porteur du gène codant la recombinase cre (Sauer, B. (1987), Mol. Cell. Biol., 7, 2087-2096) qui permet la recombinaison des séquences IoxP/IoxR et l'élimination du marqueur. Cette technique est parfaitement connue dans l'art antérieur (Hoess, R. and Abremski, K. (1984) J. Mol. Biol., 181, 351-362).

L'étape 7 est une étape standard de toute méthode de sélection de mutant qui consiste à isoler un clone sélectionné par repiquage successif jusqu'à obtention d'une souche pure.

Précisément, l'invention a pour objet une procédé d'obtention d'une souche de levure, particulièrement une souche *Yarrowia lipolytica,* mutante, n'exprimant pas le gène GUT2, caractérisé en ce que
- dans une première étape, on construit une cassette d'invalidation comprenant les séquences promotrices et terminatrices du gène GUT2 de levure (SEQ ID No 1), particulièrement de *Yarrowia lipolytica,* encadrant un gène codant un marqueur de sélection (gène de sélection), ledit gène de sélection étant lui-même encadré de part et d'autre de sa séquence par une (ou des) séquence(s) de recombinaison, lesdites séquences de recombinaison permettant une recombinaison entre elles conduisant à l'élimination dudit gène codant le marqueur de sélection ;
- dans une deuxième étape, on introduit ladite cassette d'invalidation obtenue en 1, dans une souche de levure, particulièrement une souche *Yarrowia lipolytica* ;
- dans une troisième étape, on sélectionne, parmi les souches de levure transformées à l'étape 2, une souche de levure, particulièrement une souche de *Yarrowia lipolytica,* défective pour le gène GUT2, souche ayant introduit par double recombinaison (double cross-over) le gène marqueur en lieu et place du gène GUT2, conduisant ainsi à un gène GUT2 invalidé (souche gut2::URA3) ;
- dans une quatrième étape, on vérifie l'invalidation dudit gène GUT2 dans ladite souche de levure sélectionnée à l'étape 3.

Selon une variante de l'invention, le procédé peut en outre présenter 2 étapes supplémentaires à savoir :
- une cinquième étape au cours de laquelle on transforme ladite souche sélectionnée à l'étape 4 avec un vecteur permettant l'expression d'une recombinase afin d'obtenir l'élimination du gène exprimant le marqueur de sélection ;
- une sixième étape, au cours de laquelle on isole une souche de levure défective pour le gène GUT2, et n'exprimant plus le gène marqueur, appelée □gut2.

Selon l'invention, à la première étape du procédé, on construit ladite cassette d'invalidation comprenant les séquences promotrices et terminatrices du gène GUT2 de levures, particulièrement de la souche *Yarrowia lipolytica,* selon la technique décrite par Fickers et coll. (2003, J. Microbiol. Methods 55/3:727-737). Pour cela
- dans un premier temps on synthétise, par exemple par PCR, et on purifie des fragments d'ADN comprenant soit la séquence Promotrice (P) soit la séquence Terminatrice (T) du gène GUT2 de levure, particulièrement de *Yarrowia lipolytica*, à partir d'ADN, par exemple de l'ADN génomique, de levure, particulièrement de *Yarrowia lipolytica,* avantageusement une souche sauvage de levure, encore plus avantageusement une souche W29 de *Yarrowia lipolytica,* préalablement purifiée. Lorsque l'on synthétise ces fragments par PCR, on peut utiliser toute paire d'amorce compatible, particulièrement avec de l'ADN de *Yarrowia lipolytica,* les paires G3P-P1/ G3P-P2 (SEQ ID N°2 et 3) pour la partie P et G3P-T1/ G3P-T2 (SEQ ID N°4 et 5) pour la partie T. Avantageusement, les amorces G3P-P2 et G3P-T1 peuvent comporter en outre chacune la séquence d'une site de restriction, préférentiellement un site rare, comme par exemple un site choisi parmi les méganucléases (Homing endonucléases), telles que I-Ceul, I-Scel, PI-PspI, PI-SceI, préférentiellement le site I-Scel ;
- dans un deuxième temps on solidarise par toute méthode connue en biologie moléculaire, un fragment P et un fragment T, avantageusement par l'intermédiaire du site de restriction, avantageusement le site I-Scel, pour former des fragments d'ADN P-site rare-T (appelés cassettes d'invalidation 2 ou cassettes d'invalidation GUT2-PT) ;
- dans un troisième temps on introduit au site de restriction de la cassette d'invalidation 2 un gène codant un marqueur de sélection, comme par exemple un gène associé à un phénotype d'auxotrophie ou de caractère dominant ; à titre de marqueurs d'auxotrophie, on citera par exemple URA3, LEU2, ADE2, HIS et LYS5. A titre de marqueurs dominants, on citera les gènes de résistance aux antibiotiques, de préférence le gène de résistance à l'hygromycine (HYG), préférentiellement un gène codant URA3, LEU2, ADE2, HYG, pour former des fragments d'ADN P-I-Sce I-Marqueur-I-Sce I-T (appelés cassette d'invalidation 1 ou cassette d'invalidation GUT2-PUT quand le gène marqueur est le gène URA3, cassette d'invalidation GUT2-PLT quand le gène marqueur est le gène LEU2.
- dans un quatrième temps on purifie ladite cassette d'invalidation 1 soit par digestion du plasmide GUT2-PUT ou GUT2-PLT par des enzymes de restriction adéquatement de part et d'autre de la cassette d'invalidation, soit par PCR avec les amorces P1 et T1.

Selon l'invention, à la seconde étape du procédé, on introduit la cassette d'invalidation 1 obtenue à la première étape, dans une souche de levure, particulièrement une souche *Yarrowia lipolytica,* sauvage comme par exemple une souche W29, ou avantageusement une souche de levure, particulièrement une souche *Yarrowia lipolytica,* n'exprimant pas le gène de marqueur de sélection contenu dans la cassette d'invalidation, comme par exemple une souche Po1d qui est une souche auxotrophe pour la leucine (Leu⁻) et l'uracile (Ura⁻), décrite par G. Barth et coll. (Non conventional Yeasts in Biotechnology A Handbook (Wolf, K., Ed.), Vol. 1, 1996, pp. 313-388. Springer-Verlag, Berlin, Heidelberg, New York) et répertoriée sous CLIB139 dans la collection du CIRM (Centre International de Ressources Microbiennes, préalablement intitulée Collection de Levures d'Intérêt Biotechnologique (CLIB), ou encore la souche JMY1202 (Leu-, Ura+, Δgut2PUT)] obtenue par complémentation de la défection Ura- de la souche Po1d par transformation de la souche Po1d avec la cassette d'invalidation de GUT2-PUT ou encore de la souche JMY1387 (Leu+, Ura+, Δgut2PUT)] obtenue par complémentation de la défection Leu- de la souche JMY1202 par transformation de la souche JMY1202 avec un fragment d'ADN contenant le gène LEU2.

Encore plus avantageusement à cette étape on pourra utiliser une souche de levure qui ne réalisera pas la β-oxydation des lipides, par exemple une souche qui n'exprimera pas les gènes responsables de la β-oxydation des lipides comme les gènes POX, MFE1 ou POT1, avantageusement une souche n'exprimant pas les gènes POX, à tout le moins les gènes POX2, POX3, POX4 et POX5, préférentiellement les gènes POX1, POX2, POX3, POX4, POX5 et POX6, comme par exemple les souches décrites dans la demande internationale WO 2006/064131 publiée le 22 juin 2006, préférentiellement les souches
MTLY37 (Leu+, Ura+; Δpox5, Δpox2, Δpox3, Δpox4 ::URA3),
MTLY40 (Leu+, Ura-; Δpox5-PT, Δpox2-PT, Δpox3-PT, Δpox4::ura3-41),
MTLY64 (Leu-, Ura-, Hyg+; Δpox5, Δpox2, Δpox3, Δpox4::ura3-41, leu2 ::Hyg),
MTLY66 (Leu-, Ura-; Δpox5, Δpox2, Δpox3, Δpox4::ura3-41, Δleu2),
MTLY82 (Leu-, Ura-, Hyg+; Δpox5, Δpox2, Δpox3, Δpox4::ura3-41, Δleu2, pox1::Hyg),
MTLY85 (Leu-, Ura-; Δpox5; Δpox2, Δpox3, Δpox4::ura3-41, Δleu2, Δpox1),
MTLY92 (Leu-, Ura-, Hyg+; Δpox5, Δpox2, Δpox3, Δpox4::ura3-41, Δleu2, Δpox1, pox6::Hyg),
MTLY95a (Leu-, Ura- ; Δpox5, Δpox2, Δpox3, Δpox4::ura3-41, Δleu2, Δpox1, Δpox6).

A cette étape toute méthode de transfert connue de l'art antérieur (voir "le Sambrook") peut être utilisée pour introduire la cassette d'invalidation 1 dans la souche de levure. Préférentiellement on pourra utiliser la méthode à l'acétate de lithium et au polyéthylène glycol décrite par Gaillardin et coll. (Curr. Genet. 11, 1987, 369-375).

Selon l'invention, à la troisième étape, il est possible d'utiliser toute méthode de sélection connue de l'art antérieur compatible avec le gène (ou les gènes) marqueur(s) utilisés, toute souche exprimant le gène marqueur choisi étant potentiellement une souche de levure défective en gène GUT2, URA3 ou LEU2.

Selon l'invention, à la quatrième étape, on vérifie l'invalidation dudit gène GUT2 dans ladite souche de levure sélectionnée à l'étape 3, par toute méthode compatible décrite dans l'art antérieur, préférentiellement par PCR selon la technique de Gussow et coll. (Nucleic Acids Res. 17, 1989, 4000), puis éventuellement confirmée par hybridation Southern blot (Sambrook).

Les souches mutantes obtenues à cette étape, susceptibles d'accumuler les lipides, expriment le marqueur de sélection. Il peut cependant être nécessaire de posséder des souches mutantes, susceptibles d'accumuler les lipides, mais n'exprimant pas le marqueur de sélection. Ainsi le procédé selon l'invention peut en outre comporter une cinquième et une sixième étapes visant à obtenir de telles souches mutantes, susceptibles d'accumuler les lipides, mais n'exprimant pas le marqueur de sélection.

Selon l'invention, à la cinquième étape on transforme ladite souche sélectionnée à l'étape 4 avec un vecteur permettant l'expression d'une recombinase afin d'obtenir l'élimination du gène exprimant le marqueur de sélection. A titre d'exemple il est possible d'introduire un vecteur réplicatif permettant l'expression de la recombinase cre et possédant le marqueur de sélection LEU2, comme le vecteur pRRQ2 (Fickers, op. cit.) ;

Selon l'invention, à la sixième étape, on isole une souche de levure défective en gène GUT2, et n'exprimant plus le gène marqueur utilisé pour l'invalidation du gène GUT2, et ayant éventuellement perdu le plasmide réplicatif permettant l'expression de la recombinase, par toute méthode de sélection ou d'isolement connue de l'art antérieur comme par exemple la croissance en milieu riche et ou l'étalement sur milieu riche qui ne permet plus de sélectionner pour le marqueur de sélection.

L'invention a également pour objet l'utilisation d'une souche de levure, particulièrement une souche *Yarrowia lipolytica,* mutante, capable d'accumuler des lipides, par exemple une souche selon l'invention, pour la synthèse de lipides, particulièrement des acides gras libres et des triacylglycerols. Préférentiellement, selon l'invention on utilisera une souche *Yarrowia lipolytica,* mutante, capable d'accumuler des lipides, avantageusement une souche n'exprimant plus le gène GUT2 et plus les gènes POX, très avantageusement la souche JMY1393 (CNCM 1-4169). Avantageusement selon l'invention les souches mutantes pourront être utilisée pour la production d'acides gras n-2, très avantageusement pour la production du 16:1 (n-9), l'acide (Z)-7-hexadedecenoique.

L'invention a encore pour objet un procédé de synthèse de lipides dans lequel
- dans une première étape on cultive, dans un milieu approprié, une souche de levure, particulièrement une souche *Yarrowia lipolytica,* mutante, capable d'accumuler des lipides, par exemple une souche selon l'invention ;
- dans une seconde étape on récolte les lipides produits par la culture de l'étape 1.

Outre les dispositions qui précèdent, la présente invention comprend également d'autres caractéristiques et avantages qui ressortiront des exemples et Figures qui suivent, et qui doivent être considérés comme illustrant l'invention sans en limiter la portée.

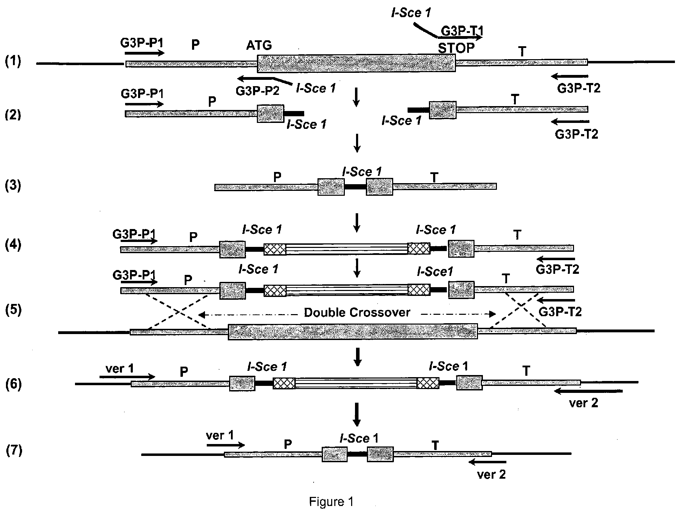

Ainsi la Figure 1 présente un schéma de la construction de mutants de levure n'exprimant pas un gène d'intérêt, ici le gène GUT2 de *Yarrowia lipolytica,* par invalidation à l'aide d'un gène marqueur.
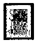 : Gène d'intérêt de levure
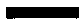 : Génome de la levure
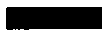 : Site de restriction rare (I-Sce 1)
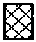 : Séquences répétées
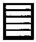 : marqueur
P : Séquences promotrices du gène d'intérêt
T : Séquences terminatrices du gène d'intérêt
G3P-P1, G3P-P2, G3P-T1, G3P-T2 : Amorces pour la synthèse des fragments P et/ou T par PCR ;
ver1, ver2 : Amorces pour la vérification de l'invalidation par PCR ;
(1) et (2) : synthèse des fragments P et T avec insertion d'un site rare aux extrémités
(3) : fragment P-site rare-T, après fusion des fragments par PCR ;
(4) : Insertion au site rare du gène marqueur encadré de séquences de : recombinaison pour obtenir la cassette d'invalidation ;
(5) : Après transformation de la souche, double crossover entre la cassette d'invalidation et le génome de la levure ;
(6) : Résultat du double cross-over : remplacement du cadre de lecture du gène d'intérêt par le gène marqueur ;
(7) : Gène d'intérêt invalidé dans la souche de levure après excision du gène marqueur par recombinaison.

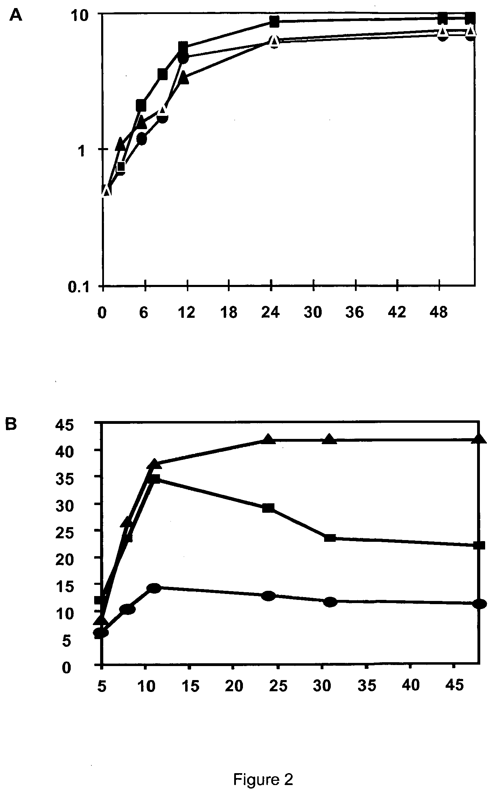

La Figure 2 présente en A la croissance des souches de type sauvage et des souches mutantes (D.O. à 600 nm) en fonction du temps (h) et en B l'accumulation totale d'acide gras, exprimée en pourcentage du poids sec de la biomasse en fonction du temps (h).
○ : souche de type sauvage ; □ : souche mutante Δgut2 ; Δ : souche mutante Δgut2 Δpox1-6)

Les résultats sont les valeurs moyennes de trois expériences indépendantes.

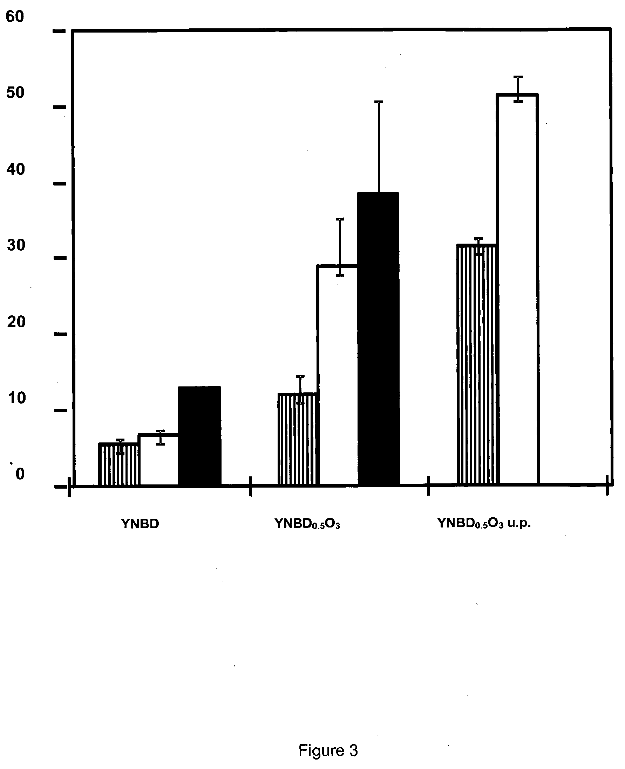

La Figure 3 présente la teneur totale en acides gras des souches de type sauvage et des souches mutantes en pourcentage du poids sec de la biomasse à la fin de la culture cellulaire (24h).

Les souches ont été cultivées sur les milieux YNBD, YNBD_{0,5}O₃ et YNBD_{0,5}O₃ u.p..

Les résultats représentent les valeurs moyennes de trois expériences indépendantes.
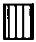 : Souche de type sauvage
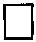 :Souche Δgut2
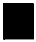 : Souche Δgut2 Δpox1-6
u. p. : acide oléique ultra-pur (>98% de pureté)

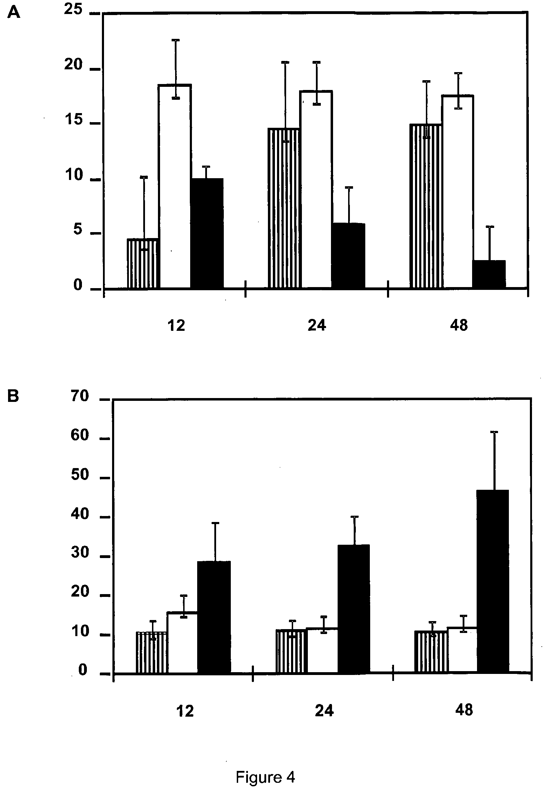

La Figure 4 présente dans la partie A. le nombre moyen des corps lipidiques en fonction du temps chez une souche sauvage et des souches mutantes et dans la partie B le pourcentage de la surface des cellules couvertes par les corps lipidiques.

Les souches ont été cultivées sur YNBD_{0,5}O₃.
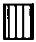 : Souche de type sauvage
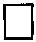 : Souche Δgut2
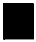 : Souche Δgut2 Δpox1-6

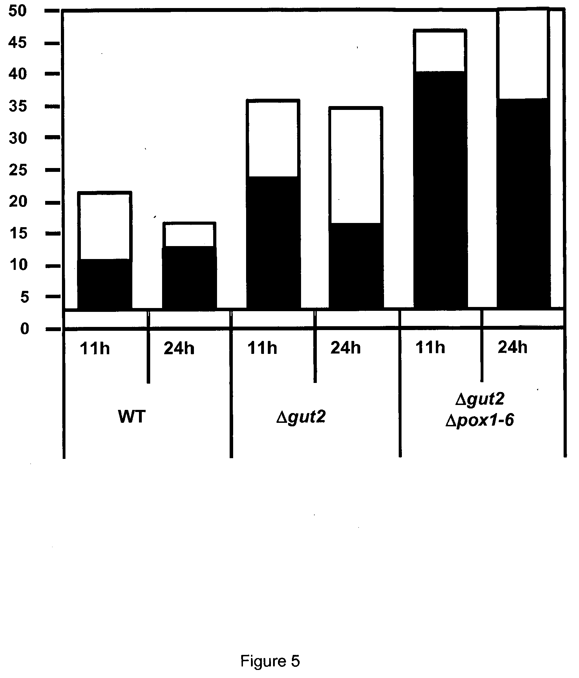

La Figure 5 présente l'accumulation des lipides totaux sous la forme de pourcentages du poids sec de la biomasse en fonction du temps (11 h et 24h), séparé en triacylglycerols et acides gras libres après culture sur le milieu YP₂D₄O₃.
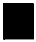 : acides gras libres 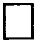
: triacylglycerols.

La Figure 6 présente l'évolution de la biomasse (•) et du pourcentage de lipides (○) produits en fonction du temps au cours de la culture de la souche sauvage W29.

La Figure 7 présente l'évolution de la biomasse (■) et du pourcentage de lipides (□) produits en fonction du temps au cours de la culture de la souche mutante Δgut2.

La Figure 8 présente l'évolution de la biomasse (▲) et du pourcentage de lipides (Δ) produits en fonction du temps au cours de la culture de la souche mutante Δgut2Δpox1-6.

La Figure 9 présente le bilan comparatif des cultures des souches
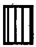 : sauvage W29
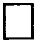 : mutante Δut2
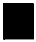 : mutante Δgut2Δpox1-6
selon :
% lip max : teneur finale en lipides exprimée en gₗᵢₚg⁻¹
R S/lip : rendement de conversion du substrat en lipides en gₗᵢₚ.g_{glc}⁻¹
P(lip) : productivité volumique de la production de lipides en gₗᵢₚ.l⁻¹.h⁻¹.

### EXEMPLES

### Matériel et méthodes

### Souches de levure et conditions de culture

Les souches de *Y. lipolytica,* utilisées dérivent de la souche sauvage *Y*. *lipolytica,* W29 (ATCC 20460).

La souche auxotrophe Po1d (Leu-, Ura-) a été décrite précédemment par Barth et Gaillardin [Nonconventional Yeasts in Biotechnology, W. K., Editor. 1996, Springer-Verlag: Berlin, Heidelberg, New York. p. 313-388)].

La souche prototrophe MTLY37 qui contient une invalidation des quatre gènes POX codant pour quatre acyl-CoA oxydases (AOX) a été décrite par Wang et collaborateurs (Wang, H.J., et coll., J Bacteriol, 1999. 181 (17): p. 5140-5148).

La souche auxotrophe MTLY40 (Ura-) ont été obtenues par transformation de MTLY37, au moyen d'un fragment de 1,5 kb portant l'allèle ura3-41, obtenu par PCR, suivie par la sélection de transformants sur milieu YNB-5FOA (Mlickova, K., et coll., Appl Environ Microbiol, 2004. 70(7): p. 3918-3924).

### Milieu de culture et conditions de croissance

Pour E. coli les conditions sont décrites dans le Sambrook et pour *Y*. *lipolytica,* dans le Barth (op. cit.).

Le milieu riche YPD, le milieu minimum glucose YNB et le milieu minimum supplémenté en casamino-acides YNBcas ont été préparés comme décrit précédemment par Mlickova, K., et coll. (op. cit.) Appl. Environ. Microbiol., 2004. 70(7): p. 3918-3924.

Le milieu minimum YNB contient 0,17% de YNBww (milieu YNB sans acides aminés et sulfate d'ammonium ; Difco, Paris, France), 0,5% de NH4CI, 0,1% d'extrait de levure (Bacto-DB) et 50 mM de tampon phosphate à pH 6,8.

Les milieux supplémentés en source de carbone sont :
- le milieu YNBD (glucose à 2%, Merck, Fontenay-sous-Bois Cedex, France) ;
- le milieu YNBG (glycérol à 2%, Merck) ;
- le milieu YNBDO (milieu supplémenté à 3% en acide oléique de Merck pur à 60%) ;
- le milieu YNBOu.p. (milieu supplémenté à 3% en acide oléique de Flucka pur à 98%) ;
- le milieu YNBO (YNBD_{0,5}O₃) utilisés pour suivre l'accumulation optimale et la remobilisation des acides gras est du milieu YNB supplémenté à 0,5% de glucose et 3% d'acide oléique ;
- le milieu YP₂D₄O₃ utilisés pour optimiser l'accumulation de lipides contient de l'extrait de levure (1%), de la protéose peptone (2%), du glucose (4%) et de l'acide oléique (3%).

De l'uracile (0,1 g / L) et de la leucine (0,2 g / L) ont été ajoutés en cas de besoin.

Pour les milieux solides, 1,5% d'agar a été ajouté.

L'acide oléique est émulsionné par sonication, en présence de 0,02% de Tween 40 (Mlickova, K., et coll. (Appl. Environ. Microbiol., 2004. 70(7): p. 3918-3924).

En règle générale, les cultures ont été réalisées comme suit :
A partir de boite YPD, une première pré-culture est inoculées dans du milieu YPD (15 ml dans des Erlenmeyers de 50 ml à 170 tr / min, à 28°C pendant 6 h). Les cellules ont été utilisées pour inoculer une pré-culture en milieu YNBD (50 ml dans un Erlenmeyer de 500 ml à 170 tr / min, à 28°C, pendant une nuit).

Pour la culture, des cellules en croissance exponentielle ont été récoltées par centrifugation au lendemain de la pré-culture, et resuspendue dans du milieu YNB frais à une densité optique à 600 nm de 0,5.

Pour déterminer la croissance des cellules, les cultures ont été centrifugées à 10000xg pendant 10 min, et le culot de cellules a été lavé deux fois avec des volumes égaux de solution SB (9 g / L de NaCl - 0,5% BSA).

La biomasse produite a été déterminée en mesurant la densité optique à 600 nm. et par l'estimation du poids sec des cellules après chauffage à 80°C pendant 24 h.

### Techniques générales de génétique

Les techniques générales de génétique moléculaire ont été utilisées comme décrit dans le Sambrook.

Les enzymes de restriction utilisées proviennent de Eurogentec SA (Liège, Belgique).

L'ADN génomique de levure (sauvage ou transformées) a été préparé tel que décrit par Querol et coll. (Appl. Environ. Microbiol., 1992. 58(9): p. 2948-2953).

Les amplifications par PCR ont été réalisées sur un thermocycleur Eppendorf 2720, avec soit de l'ADN polymérase Taq (Promega, Madison, WI, USA) soit de l'ADN polymérase Pfu (STRATAGENE, La Jolla, Californie).

Le tableau 2 suivant décrit les amorces utilisables selon l'invention :

**Tableau 2**

| Amorces | SEQ ID N° | Séquence (5'→ 3')^{a} | site de restriction, introduit |
|---|---|---|---|
| G3P-P1 | 2 | GCAGATCCACTGTCAAGCCG | |
| G3P-P2 | 3 | | I-Scel |
| G3P-T1 | 4 | | I-Scel |
| G3P-T2 | 5 | GCAGCCAGCAGCACGTAGTAG | |
| G3P-ver1 | 6 | GAATGACGGGGGCAACGCAG | |
| G3P-ver2 | 7 | CAGCAGCCACAAATAGCAGACTGCC | |
| LEU2-P1 | 8 | | Xbal |
| LEU2-P2 | 9 | | I-Scel |
| LEU2-T1 | 10 | | I-Scel |
| LEU2-T2 | 11 | | Hindlll |
| POX1-P1 | 12 | CATGGAGTGGATCGCTCGAGGACG | |
| POX1-P2 | 13 | | I-Scel |
| POX1-T1 | 14 | | I-Scel |
| POX1-T2 | 15 | CGGCAGTGGCTCACCAAGC | |
| POX1 ver1 | 16 | ATCCAGACCTCCAGGCGGG | |
| POX1ver2 | 17 | GCTGCGTCTCAATCTGGCGAATG | |
| POX6-P1 | 18 | CCAAGCTCTAAGATCATGGGGATCCAAG | |
| POX6-P2 | 19 | | I-Scel |
| POX6-T1 | 20 | | I-Scel |
| POX6-T2 | 21 | ATCTCGAGATTGGTCCCCTCAAACACAC | |
| POX6Ver1 | 22 | GCTCAAGAAGGTAGCTGAGTC | |
| POX6ver2 | 23 | CATTAAGTGTCAGATCAGCTCGC | |

| | | | |
|---|---|---|---|
| ^{a} les séquences soulignées correspondent aux sites de restriction introduits. | | | |

Les amorces P1, P2, T1 et T2 servent à la construction des cassettes d'invalidation. Les amorces ver1 et ver2 servent à la vérification de l'invalidation des gènes par PCR.

Les fragments de PCR ont été purifiés au moyen du kit de purification QIAGEN (Qiagen, Hilden, Allemagne) et les fragments d'ADN récupérés à partir de gels d'agarose en utilisant un kit QIAquick Gel Extraction (Qiagen, Hilden, Allemagne).

L'ensemble de programmes Staden (Dear, S. et Staden, R. A sequence assembly and editing program for efficient management of large projects. Nucleic Acid Res. 19, 3907-3911 (1991)) a été utilisé pour l'analyse des séquences.

La transformation des cellules de levure a été réalisée selon la méthode à l'acétate de lithium décrite dans Le Dall, M.T. et coll. (Curr Genet, 1994. 26(1): p. 38-44).

### Suppression des gènes GUT2, LEU2, POX1 ou POX6, expression de la recombinase Cre et excision du marqueur

Les cassettes de suppression ont été générées par amplification PCR selon Fickers et coll. (op. cit.) (J.Microbiol. Methods, 2003, v.55, issue 3 p. 727-737).

Le fragment PT a été cloné dans le plasmide PCR4 RBlunt-TOPO (vecteur TOPO-pCR4 ; Invitrogen Corp., Carlsbad, CA) pour obtenir un premier plasmide nommé JME743.

Le marqueur URA3 a été ensuite introduit au site I-SceI du plasmide JME743 pour obtenir un second plasmide nommé JME744 contenant la cassette d'invalidation gut2-PUT.

Pour le gène LEU2 les paires d'amorce LEU2-P1 / LEU2- P2 (SEQ ID N°8 et 9) et LEU2-T1 / LEU2-T2 (SEQ ID N°10 et 11) ont été utilisées. P1 a été conçu pour mettre en place un site de restriction Xbal à l'extrémité 5' du fragment P, alors que T2 a été conçu pour mettre en place un site de restriction HindIII.

Après amplification par PCR, le fragment PT a été digérée par Xbal et HindIII et cloné aux sites correspondant du plasmide pDRIVE (Qiagen, Les Ulis, France) pour obtenir un troisième plasmide nommé JME641. Puis le marqueur HPH (HYG) a été introduit pour obtenir un quatrième plasmide nommé JME651. Le marqueur HPH (HYG), originaire d'un plasmide d'Escherichia coli, permet la résistance à l'hygromycine B (GRITZ & DAVIES, Yeast, vol.8, p : 667-668, 1992 ;).

Pour les gènes POX1 ou POX6, les séquences PT ont été amplifiées avec leurs paires d'amorce respectives POXn-P1/POXn-P2 et POXn-T1/POXn-T2 (voir le tableau 2) et clonées en extrémités franches dans le plasmide Bluescript KS⁺ (STRATAGENE).

Après transformation dans E. coli DH5α et culture sur LB Agar Xgal des colonies blanches ont été sélectionnées.

Les souches MTLE34 et MTLE35 contenaient les plasmides porteurs des cassettes POX1-PT et POX6 PT, respectivement.

Les souches MTLE36 et MTLE37 contenaient les plasmides POX1-PHT et POX2-PHT, respectivement.

Les cassettes d'invalidation (PUT et PHT), obtenues par PCR, ont été utilisées pour la transformation par la méthode à l'acétate de lithium. Les transformants ont été sélectionnés sur milieu YNBcasa et YPDH, respectivement.

L'invalidation a été vérifiée par PCR en utilisant des paires d'amorces ver1/ver2 (voir tableau 2) et l'excision du gène marqueur à été réalisée conformément à Fickers et Coll. (op. cit.) (J.Microbiol. Methods, 2003, v.55, issue 3 p. 727-737)

### Microscopie optique

Pour les expériences en microscopiques optique 10 mL d'une culture de levure en croissance ont été pré-fixés par 1,34 ml de solution de formaldéhyde (tampon phosphate 50 mM, pH 6,8; 0,5 mM MgCl2; 4,8% de formaldéhyde) et incubés pendant 1 h à 28°C sous agitation à 250 tr / min. Les cellules pré-fixées ont été récoltées, remises en suspension à une densité optique À 600 nm de 2,5 dans la solution de formaldéhyde et incubé pendant 5 h à température ambiante. Puis les cellules ont été lavées deux fois avec de tampon phosphate 50 mM (pH 6,8) et stockées dans du tampon phosphate 0,1 M (pH 7,5) à une densité optique À 600 nm de 2,5 à 4°C jusqu'à observation en microscopie optique.

### Microscopie par fluorescence

Pour la visualisation des corps lipidique, du rouge du Nil (1mg/ml solution dans l'acétone; Bioprobe moléculaire, Montluçon France) a été ajouté à la suspension cellulaire (1/10 v/v) et suivi d'une incubation 1 h à température ambiante.

Les cellules ont été récoltées, lavées deux fois avec de l'eau distillée et remises en suspension dans du tampon phosphate 50 mM (pH 6,8) à une densité optique à 600 nm de 2,5. L'étude a été réalisée avec un microscope optique Olympus BX 51 avec un objectif X 100 à bain d'huile. Pour les enregistrements le logiciel Photometrics CoolSNAP 2048 x 2048- element array, 7.4 x 7.4-µm pixel pitch (Roper Scientific, Inc Photometrics, PVCAM) a été utilisé.

### Détermination des lipides

Les lipides d'une culture présentant une densité optique équivalente à 10 de DO ont été extraits soit par la procédure de Folch et coll. (Folch, J. et coll., J Biol Chem, 1957. 226(1) : p. 497-509) pour l'analyse TLC ou directement convertis en leurs esters méthyliques en utilisant des cellules lyophilisés selon Browse, J., et coll. (Anal Biochem, 1986. 152(1) : p. 141-5) pour l'analyse par chromatographie en phase gazeuse.

La transméthylation complète a été vérifiée par une méthode au BF₃ dans le méthanol (Athenstaedt, K., et coll., J Bacteriol, 1999. 181(20): p. 6441-8) et sur plaques TLC.

L'analyse par chromatographie en phase gazeuse des esters méthyliques d'acides gras a été réalisée à l'aide d'un Varian 3900 équipé d'un détecteur à ionisation de flamme et une colonne Varian FactorFour vf-23ms, (3pA à 260°C [30m, 0,25, 0,25 µm]).

Les acides gras ont été identifiés par comparaison à des esters méthyliques d'acides gras normés (Fatty Acid Methyl Ester, FAME, Cayman, Supelco, Sigma (France)) et quantifiés par la méthode de l'étalon interne en ajoutant 50 µg de C17:0 commercial (Sigma).

### Analyse des classes de lipides

Les lipides totaux ont été fractionnés en triacylglycérol et acides gras libres pour la quantification des lipides en utilisant une colonne Isolute SPE Aminopropyl (IST, France, Paris, France). Le conditionnement de la colonne a été réalisé 3 fois avec 3 ml d'hexane normal à débit normal. 1 ml de l'ensemble des lipides extraits par la méthode de Folch dans du CHCl₃ a été chargé dans la colonne et la fraction des lipides neutres a été recueillie.

L'élution totale des lipides neutres a été réalisée par lavage de la colonne 3 fois avec 3 ml de CHCl₃/Isopropanol (2/1). La fraction acides gras libres a été recueillie en lavant la colonne 3 fois avec 3 ml de ET₂O/acide acétique 2% avec un débit normal. Le solvant des fractions a été évaporé sous flux d'azote direct et la transméthylation a été suivie pour analyse par chromatographie en phase gazeuse (Laffargue, A. et coll., Plant Physiol Biochem, 2007. 45(3-4): p. 250-7).

Des plaques TLC ont été utilisées pour les extractions de vérification. L'efficacité de la procédure a également été vérifiée par comparaison des profils de chromatographie en phase gazeuse des échantillons fractionnés ou non.

### Séparation des lipides par TLC

Des plaques TLC (silice G60, 20*20 cm, épaisseur 0,25 mm) (Merck, Allemagne) ont été utilisées. Les différentes classes de lipides ont été séparées en utilisant un système de solvant à double de développement : Système A (demi-plaque de migration): éther de pétrole/éther éthylique/ acide acétique: 20/20/0,8 (v/v/v); Système B (toute la plaque de migration): Ether de pétrole/Et₂O 49/1 (v/v). Une solution à 5% d'acide phosphomolybdique a été pulvérisée sur les plaques et les bandes de lipides ont été révélées au bout de 10 min à 105°C.

### Exemple 1 : Obtention de la souche mutante Δgut2

### Préparation de l'ADN génomique de la souche sauvage Yarrowia lipolytica, W29

La souche sauvage *Yarrowia lipblytica,* W29 est mise en culture sur boite YPD solide pendant 1 jour à 28°C.

À partir d'une colonie isolée, une culture YPD liquide (5 ml) est incubée 40 à 48 heures à 28°C sous agitation (170 rpm).

Les cellules de 3 ml de culture sont récoltées par centrifugation à 13000 rpm pendant 5 min.

Le culot est alors suspendu dans 500 µl de Tampon Sorbitol (Sorbitol 1 M, Tris-HCl 0,1 M ; pH 8 ; EDTA 0,1M).

Après ajout de 50 µl de solution zymolyase à 3 mg/ml (Zymolyase 100T, Seikagaku Corp. Coger) et de 50 µl de β-mercaptoéthanol (0,28 M), les cellules sont incubées à 37°C pendant 1 h.

Les cellules sont alors récoltées par centrifugation et remises en suspension dans 500 µl de TE (50 mM Tris-HCl ; pH 8,0 ; 20 mM EDTA).

Après ajout de 50 µl de SDS 10%, elles sont incubées pendant 20 min à 65°C. Ensuite 200 µl de solution d'acétate de potassium 5 M sont ajoutées.

Les cellules sont conservées dans la glace pendant 30 min, avant centrifugation (5 min, 13000 rpm).

Le culot est re-suspendu dans 700 µl d'isopropanol, centrifugé 5 min à 13000 rpm et lavé avec 500 µl éthanol 70%.

Après élimination du surnageant, 400 µl de TE-Rnase A (TE + 100 µg/ml de RNase A) sont ajoutées et les cellules sont incubées 15 min à 1 h à 37°C.

L'ADN est précipité à l'aide de 40 µl acétate de potassium (2,5 M ; pH 5,2) et 1 ml d'éthanol 100%. On centrifuge 5 min à 13000 rpm et on élimine le surnageant.

Un lavage avec 700 µl d'éthanol 70% est effectué. Après séchage du culot on récupère l'ADN génomique dans 100 µl d'eau.

### Synthèse de l'ADN du gène GUT2

La synthèse de l'ADN du gène GUT2 est réalisée par PCR (Eppendorf 2720 thermocycler) dans un microtube de 0,5 ml.

Le mélange réactionnel est composé de 10 ng d'ADN génomique de la souche sauvage *Yarrowia lipolytica,* W29 obtenue en exemple 1 en présence de 50 pmoles de chaque amorce correspondant dans le régions en amont (P1) et en aval (T2) respectivement de l'ORF du gène GUT2, 200 µM de chaque dNTP, 1,5 mM MgCl2, 2,5 U de Pfu (STRATAGENE, La Jolla, CA) et le tampon fourni avec l'enzyme à la concentration indiqué dans un volume final de 50 µl.

La PCR se déroule en 30 cycles d'amplification successifs.

Chaque cycle est composé d'une étape de dénaturation de l'ADN pendant 1 min à 94°C, une étape d'hybridation des 2 amorces spécifiques pendant 30 secs à 57°C et une étape d'élongation de la copie d'ADN à 72°C.

Les fragments ADN se sont récupérés à partir de gels d'agarose en utilisant le kit QIAquick Gel Extraction Kit (Qiagen, Hilden, Germany).

### Synthèse des fragments P et T du gène GUT2 et constitution du fragment P-T

La cassette PT (Promoteur-Terminateur) est obtenue par deux étapes d'amplification PCR.

Le promoteur et le terminateur sont situés respectivement en amont et en aval de l'ORF du gène GUT2.

Ces deux régions, d'environ 1 kb, sont amplifiées à partir de l'ADN génomique de la souche sauvage *Yarrowia lipolytica,* W29 avec, respectivement, les couples d'amorces P1/P2 et T1/T2.

Les amorces P2 et T1 contiennent le site rare de reconnaissance de la méganucléase I-Scel.

Les fragments PCR obtenus, P-I-Scel et I-Scel-T, sont ensuite réunis par ligation puis utilisés comme matrice pour l'amplification PCR de la cassette P-I-Scel-T (PT) en utilisant les amorces P1, T2.

Cette cassette est ensuite clonée dans le plasmide pCR4-Blunt TOPO (INVITROGEN).

Après transformation dans E. coli, le vecteur pCR4-TOPO-PT appelé GUT2-PT (JME743) est isolé.

### Obtention du gène URA3 :

La cassette I-Scel URA3 est obtenue par digestion par I-Scel du plamide KS-URA3 (JME507 ; Fickers et col. 2003 Op. cit.).

Le fragment I-Scel-URA3 et purifié sur gel d'agarose.

Insertion dans le fragment P-T :
Le plasmide portant la cassette GUT2-PT (JME743) est digérés par I-Scel.

Après déphosphorylation du produit de digestion du plasmide PT, une ligature entre celui-ci et le marqueur de sélection I-Scel-URA3 est réalisée.

Les plasmides résultants présentent le module P-URA3-T et sont appelés JME744 (GUT2-PUT).

La cassette d'invalidation PUT est généré par amplification PCR en utilisant le plasmide GUT2-PUT et les amorces P1/T2.

Le fragment PCR est ensuite utilisé pour l'invalidation du gène GUT2 par transformation de *Y. lipolytica.*

### Transformation de la souche de levure :

La méthode utilisée pour la transformation de *Y. lipolytica,* repose sur l'utilisation de l'acétate de lithium pour perméabiliser les cellules (Gaillardin et al., 1985).

Les cellules (souche Po1d) sont cultivées à 28°C dans 20 mL de milieu YPD complémenté de tampon citrate 50 mM pH 4.

Quand la culture présente une concentration cellulaire proche de 10⁸ cellules/ ml, elle est centrifugée.

La compétence est acquise après incubation des cellules, re-suspendues dans 20 ml d'acétate de lithium 0,1 M pH 6, pendant 1 heure à 28°C sous agitation (100 rpm).

Les cellules sont ensuite centrifugées pendant 2 min à 2000 rpm et elles sont reprises dans la même solution afin d'obtenir une concentration de 5.10⁸ cellules/ ml.

La transformation s'effectue en ajoutant 5 µL d'ADN entraîneur (poisson) et 10 µL d'ADN à transformer (cassette GUT2-PUT) à 100 mL de cellules compétentes.

Le mélange est incubé sans agitation à 28°C, puis 0,7 ml de PEG 4000 (40 %) en acétate de lithium (0,1 M ; pH 6) sont ajoutés.

Ce mélange est incubé sous agitation (250 rpm) pendant une heure à 28°C. Puis, les cellules subissent un choc thermique de 1 min à 39°C.

Le mélange est dilué par addition de 1,2 ml de solution d'acétate de lithium 0,1 M pH 6.

### Sélection et purification des souches de levures mutantes exprimant le gène URA3.

La transformation est étalée à raison de 200 µL par boite de milieu YNB - casaminoacides (milieu ne contenant pas d'uracile).

Les transformants apparaissent entre 3 et 5 jours d'incubation à 28°C.

Un deuxième étalement à partir d'une colonie isolée est effectué dans le milieu sélectif afin de purifier les transformants.

### Vérification de l'invalidation du gène GUT2

La vérification de la délétion du gène est réalisée par PCR en utilisant les amorces ver1 et ver2 spécifiques du gène GUT2 et situés à l'extérieur de la cassette d'invalidation.

La taille du fragment amplifié permet la vérification de l'invalidation du gène GUT2 comparé à la taille du fragment amplifié chez la souche sauvage avec les mêmes amorces.

### Excision du gène URA3

Le marqueur URA3ex utilisée peut s'exciser par expression de la recombinasse Cre.

Les cellules de levure sont transformées par le plasmide pRRQ2 (hp4d-cre, yILEU2) et les transformant Leu+ sont sélectionnée sur milieu sans leucine.

Ensuite, pour permettre la perte du vecteur d'expression de la recombinasse Cre, les transformants Leu+ sont cultivées en milieu YPD pendant 12h à partir d'une préculture YPD diluée à 1 /1000.

Les cellules sont ensuite étalées sur milieu YNB.

Les clones Ura- (excision du marqueur) et Leu- (perte du plasmide pQRR2) sont sélectionnés.

L'excision du marqueur est confirmée par PCR avec les amorces ver1/ver2.

**Tableau 3**

| Étape | Opérations de transformation | | |
|---|---|---|---|
| | Mutant à transformer | Cassette de transformation | Mutant transformé |
| 1 | Po1d, Leu-, Ura-, | Cassette PUTgut2, sélection Ura+ | JMY1202, Leu-, Ura+, Δgut2 |
| 2 | JMY1202, Leu-, Ura+, Δgut2 | Fragment génomique contenant le gène LEU2 | JMY1387, Leu+, Ura+, Δgut2 |

| | | | |
|---|---|---|---|
| Leu+/ Leu- : souche exprimant / n'exprimant pas le gène LEU2. Ura+/ Ura- : souche exprimant / n'exprimant pas le gène URA3. | | | |

### Exemple 2 : Obtention des souches de levure Yarrowia lipolytica mutantes MTLY40, MTLY64, MTLY66, MTLY82, MTLY85, MTLY92, MTLY95a et JMY 1393

En reprenant le protocole de l'exemple 1 et suivant les spécifications données au tableau 4 ci-après on construit les souches de levure *Yarrowia lipolytica,* mutantes MTLY40, MTLY64, MTLY66, MTLY82, MTLY85, MTLY92, MTLY95a et JMY 1393.

La souche MTLY37 (Leu+, Ura+, Δpox2-5), précédemment décrite dans demande internationale WO 2006/064131, est une souche phototrophe pour la leucine et l'uracile.

**Tableau 4**

| Étape | Opérations de transformation | | |
|---|---|---|---|
| | Mutant à transformer | Cassette de transformation | Mutant transformé |
| 1 | MTLY37, Leu+ Ura+ Δpox5-PT, Δpox2-PT, Δpox3-PT, Δpox4-PUT | Fragment de PCR ura3-41, sélection 5FOA | MTLY40, Leu+, Ura-, Δpox5-PT, Δpox2-PT, Δpox3-PT, Δpox4-Pura3-41T |
| 2 | MTLY40, Leu+, Ura-, Δpox5-PT, Δpox2-PT, Δpox3-PT, Δpox4-Pura3-41T | Cassette PHTleu2, sélection hygromycine | MTLY64, Leu-, Ura-, Hyg+, Δpox5-PT, Δpox2-PT, Δpox3-PT, Δpox4-Pura3-41T, leu2-PHT |
| 3 | MTLY64, Leu-, Ura-, Hyg+, Δpox5-PT, Δpox2-PT, Δpox3-PT, Δpox4-Pura3-41T, leu2-PHT | Vecteur pRRQ2, sélection Leu+, vérification Hyg-, perte du plasmide pRRQ2 sur YPD, isolement de Leu- | MTLY66, Leu-, Ura-, Δpox5-PT, Δpox2-PT, Δpox3-PT, Δpox4-Pura3-41T, Δleu2 |
| 4 | MTLY66, Leu-, Ura-, Δpox5-PT, Δpox2-PT, Δpox3-PT, Δpox4-Pura3-41 T, Δleu2 | Cassette PHTpox1, sélection hygromycine | MTLY82, Leu-, Ura-, Hyg+, Δpox5-PT, Δpox2-PT, Δpox3-PT, Δpox4-Pura3-41T, Δleu2, pox1-PHT |
| 5 | MTLY82, Leu-, Ura-, Hyg+, Δpox5-PT, Δpex2-PT, Δpox3-PT, Δpox4-Pura3-41T, Δleu2, pox1-PHT | Vecteur pRRQ2, vérification Hyg-, perte du plasmide pRRQ2 sur YPD | MTLY85, Leu-, Ura-, Δpox5-PT, Δpox2-PT, Δpox3-PT, Δpox4-Pura3-41T, Δleu2, Δpox1 |
| 6 | MTLY85, Leu-, Ura-, Δpox5-PT, Δpox2-PT, Δpox3-PT, Δpox4-Pura3-41 T, Δleu2, Δpox1 | Cassette PHTpox6, sélection hygromycine | MTLY92, Leu-, Ura-, Hyg+, Δpox5-PT, Δpox2-PT, Δpox3-PT, Δpox4-Pura3-41T, Δleu2, Δpex1, pox6-PHT |
| 7 | MTLY92, Leu-, Ura-, Hyg+, Δpox5-PT, Δpex2-PT, Δpox3-PT, Δpox4-Pura3-41T, Δleu2, Δpox1, pox6-PHT | Vecteur pRRQ2, vérification Hyg-, perte du plasmide pRRQ2 sur YPD | MTLY95a, Leu-, Ura-, Δpox5-PT, Δpox2-PT, Δpox3-PT, Δpox4-Pura3-41T, Δleu2, Δpox1, Δpox6 |
| 8 | MTLY95a, Leu-, Ura-, Δpox5-PT, Δpox2-PT, Δpox3-PT, Δpox4-Pura3-41T, Δleu2, Δpox1, Δpox6 | Cassette PUTgut2, Cassette PUTgut2, sélection uracile | JMY1351, Leu-, Ura+, Δpox5-PT, Δpox2-PT, Δpox3-PT, Δpox4-Pura3-41T, Δleu2, Δpox1, Δpox6, Δgut2-PUT |
| 9 | JMY1351, Leu-, Ura+, Δpox5-PT, Δpox2-PT, Δpox3-PT, Δpox4-Pura3-41T, Δleu2, Δpox1, Δpox6, Δgut2-PUT | Fragment génomique contenant le gène LEU2 | JMY 1393, Leu+, Ura+, Δpox5-PT, Δpox2-PT, Δpox3-PT, Δpox4-Pura3-41T, Δpox1, Δpox6, Δgut2-PUT |

| | | | |
|---|---|---|---|
| Leu+/ Leu- : souche exprimant / n'exprimant pas le gène LEU2. Ura+/ Ura- : souche exprimant / n'exprimant pas le gène URA3. Hyg+/Hyg- : souche exprimant / n'exprimant pas le gène HYG (résistance à l'hygromycine). Δpoxn-PT : souche n'exprimant pas le gène POXn, par invalidation à l'aide du fragment PT du gène n de POX.; Δpox4-PUT : souche n'exprimant pas le gène POX4, par invalidation à l'aide du fragment PUT contenant le gène URA3. | | | |

Fragment de PCR ura3-41 : (Mauersberger S. et coll. : J. Bacteriol. 183:5102-5109). L'amplification par PCR d'un fragment de 4650 bp englobant le gène URA3 délété de 10 paires de base (allèle ura3-41) a été réalisée sur l'ADN génomique de *Y*. *lipolytica,* JMY322 en utilisant les amorces
Ura3-dis1 (5'-GGGGTGACACTGCACTATTGGTTTG-3') et
Ura3-dis2 (5'GCGTTACTGAGAGGCAGAGTACATG-3').

Le milieu réactionnel de la PCR est constitué de 0,5 µL DNA, 1 µl d'amorce ura3-dis1 et ura3-dis2 100 pM, 4 µl de dNTP 2,5 mM, 1 unité de DNA polymérase (Ex taq, Takara), 5 µl de tampon (10 x taq buffer, Takara) et 39 µl d'eau. Le programme de température est de 94 °C 2 min puis 30 cycles de 30 sec à 94 °C, 45 sec à 55°C, 5 min. à 72 °C.

Sélection 5FOA : Sélection sur un milieu contenant du 5-FOA (5-fluoroorotic acid, permet de sélectionner les souches Ura-.

Δpox4-Pura3-41T : souche n'exprimant pas le gène POX4, par invalidation à l'aide du fragment PUT contenant le marqueur URA3 qui à été converti en allèle ura3-41 par transformation avec le fragment ura3-41 du gène URA3 et qui confère le phénotype Ura-.

Cassette PHTleu2 : cassette d'invalidation comprenant un gène de résistance à l'hygromycine (H) inséré entre les fragments P et T du gène LEU2.

leu2 / Δleu2-PHT souche n'exprimant pas le gène LEU2, par invalidation à l'aide du fragment PHT contenant le gène de résistance à l'hygromycine Hyg.

Hyg : marqueur de sélection Hyg (H) permettant la résistance à l'hygromycine.

Vecteur pRRQ2 : Vecteur réplicatif permettant l'expression de la recombinase cre et contenant le marqueur de sélection LEU2 (Fickers, op. cil.).

Cassette PHTpox1 : cassette d'invalidation comprenant un gène de résistance à l'hygromycine (H) inséré entre les fragments P et T du gène POX1.

Cassette PHTpox6 : cassette d'invalidation comprenant un gène de résistance à l'hygromycine (H) inséré entre les fragments P et T du gène POX6.

YPD : Milieu riche YPD pour perdre le plasmide réplicatif.

Δgut2-PUT : souche n'exprimant pas le gène d'intérêt GUT2 et exprimant le marqueur de sélection URA3 inséré dans une cassette entre les fragments P et T.

Dans les exemples suivants, l'influence de la délétion du gène GUT2 dans différentes conditions de culture et de composition du milieu sur l'accumulation de lipide par la levure (niveau d'accumulation de lipide, vitesse d'accumulation et type de lipide accumulé) a été déterminée.

On a ainsi constaté qu'une souche contenant la délétion Δgut2 accumule plus rapidement les lipides et que la teneur en lipide est plus élevée qu'une souche ne contenant pas cette délétion.

### Exemple 3 : Comparaison de l'accumulation de lipide par le mutant Δgut2 (JMY1202) de l'exemple 1 et le mutant Δgut2Δpox1-6 (JMY 1393) de l'exemple 2 à partir de glucose

Une préculture du mutant Δgut2 de l'exemple 1, conservée sur milieu gélosé de composition : extrait de levure 10 g.l⁻¹; peptone 10 g.l⁻¹; glucose 10 g.l⁻¹; Agar 20 g.l⁻¹, est effectué grâce à un ensemencement qui fournit une absorbance initiale à 600 nm du milieu de préculture voisine de 0,50.

La préculture est conduite sous agitation orbitale (200 tours.mn⁻¹) pendant 6 h à 28°C dans une fiole à ailettes de 50 ml contenant 5 ml de milieu constitué de 10 g.l⁻¹ d'extrait de levure, 10 g.l⁻¹ de peptone et 10 g.l⁻¹ de glucose.

Le milieu utilisé pour la culture est composé d'eau désionisée, d'extrait de levure à 10 g.l⁻¹; de YNB à 1,7 % g.l⁻¹ à 20 g.l⁻¹; du NH4CI à 5 ml.l⁻¹ et de glucose à 20 g.l⁻¹. Le pH de la culture est maintenu grâce à l'ajout de tampon phosphate à pH 6,8 à une concentration finale de 50 mM.

L'ensemencement de la culture est effectué avec l'équivalent d'une absorbance initiale à 600 nm de 0,5 fournit par le milieu de préculture.

La culture est conduite à 28°C dans des fioles à ailettes de 500ml avec 50 ml de milieu à une vitesse d'agitation de 200 tours.mn⁻¹.

À 11, 24 et 48 heures de culture on effectue des prélèvements de biomasse correspondante à 10 DO à 600nm. On élimine le surnageant par centrifugation. On effectue 2 lavages de la biomasse cellulaire avec du NaCl à 9 g.l⁻¹. La biomasse est ensuite lyophilisée et les acides gras sont extraits par la méthode de Folch et al. (J. Biol. Chem., 1957. 226(1) : p. 497-509).

La composition en acides gras accumulés est déterminée par chromatographie en phase gazeuse sur une colonne Varian Factor Four vf-23ms, équipé avec un détecteur d'ionisation de flamme, après transméthylation des acides gras selon la méthode de BF₃ en méthanol décrite par Athenstaedt et Coll. (J. Bacteriol., 1999. 181(20): p. 6441-8).

Une transméthylation directe sans effectuer d'extraction des lipides auparavant selon la méthode décrite par Browse et coll. (Anal. Biochem., 1986. 152(1): p. 141-5) a également été réalisée.

La variation de la température du four du chromatographe est programmée de 150°C à 260°C à raison de 8°C par minute.

Les résultats montrent une production maximale de lipides de 420 mg.l⁻¹ après 24 heures.

Les lipides totaux représentent 12,9% du poids sec (w/w).

### Exemple 4 : Comparaison de l'accumulation de lipide par le mutant Δgut2 (JMY1202) de l'exemple 1 et le mutant Δgut2Δpox1-6 (JMY1393) de l'exemple 2 à partir de l'acide oléique

On répète l'exemple 3 avec la souche (JMY1393) en présence d'acide oléique (3%) auquel on ajoute du glucose (0,5%).

Les résultats montrent une production de lipides 1,56 g.l⁻¹ à 11 h de culture et de 1,77g.l⁻¹ à 24 h de culture.

Les lipides représentent à 11 h 38,51% du poids sec et à 24h 42% du poids sec.

### Exemple 5 : Répartition des lipides accumulés sur milieu oléique en acides gras libres et triacylglycérols

À partir du milieu de culture à 24 h de l'exemple 4, une extraction des lipides totaux par la méthode Folch est réalisée.

Les lipides ainsi purifiés ont ensuite été séparés en acides gras libres (FFA) et en triacylglycérols (TAG) par extraction en phase solide à l'aide d'une colonne SPE Aminopropyl column (IST-France) selon le protocole de Laffargue et coll. (Plant Physiol. Biochem., 2007. 45(3-4): p. 250-257).

La composition de chaque fraction de lipides est déterminée par chromatographie en phase gazeuse et est résumée dans le Tableau 5 ci-dessous (les résultats sont exprimés en % des lipides totaux).

**Tableau 5 :**

| Mutant Lipides | | | Δgut2Δpox1-6 | |
|---|---|---|---|---|
| | | | TAG | FFA |
| C16:0 | | | 4,45 | 6,56 |
| C16:1 (n-9) | | | 0,25 | 0,06 |
| C16:1(n-7) | | | 0,12 | 0,09 |
| C18:0 | | | 0,72 | 1,94 |
| C18:1 (n-9) | | | 63,58 | 70,31 |
| C18:2(n-6) | | | 24,93 | 17,26 |
| Totales: | | | 94,04 | 96,22 |

### Exemple 6 : Comparaison de l'accumulation de lipides par une souche sauvage GUT2 et les mutants Δgut2 (JMY1202) et Δgut2Δpox1-6 (JMY 1393) des exemples 1 et 2 à partir d'un milieu contenant de l'acide oléique et du glucose

On répète le processus de l'exemple 3 avec un milieu de culture composé d'eau désionisée, d'extrait de levure à 10 g.l⁻¹; de protéose peptone à 20 g.l⁻¹; du NH₄Cl à 5ml.l⁻¹, de glucose à 40 g.l⁻¹ et d'acide oléique à 30g.l⁻¹ (pureté 65%, Sigma). Le pH de la culture est maintenue grâce à l'ajout de tampon phosphate à pH 6,8 à une concentration finale de 50mM.

Dans ces conditions
- la quantité de lipides accumulés s'établit comme suit :

| | 24h | 48h | 72h |
|---|---|---|---|
| Δgut2 | 2,55 g.l⁻¹ | 6,71 g.l⁻¹ | 8,24 g.l⁻¹ |
| Δgut2 Δpox1-6 | 4,12 g.l⁻¹ | 7,27 g.l⁻¹ | 9,1 g.l⁻¹ |

- le pourcentage du poids sec en lipides s'établit comme suit :

| | 11h | 24h | 48h | 72h |
|---|---|---|---|---|
| gut2 | 7,2% | 10,6% | - | - |
| Δgut2 | 12,0% | 18,0% | 23,0% | 20,0% |
| Δgut2 Δpox1-6 | 25,0% | 29,0% | 35,0% | 35,0% |

L'ensemble des résultats obtenus montre l'intérêt à utiliser des souches mutantes de levure selon l'invention pour produire des lipides.

### Exemple 7 : Analyse comparative de la production de lipide par les souches sauvages (W29), delta gut2 (dérivée de la JMY1202 rendue prototrophe, JMY1387) et delta gut2 delta Pox1-6 (JMY1393)

Les cultures en réacteur sont réalisées en mode discontinu alimenté (fed-batch) dans un fermenteur (Braun Biostat E) de 20 litres utiles.

Les différentes fonctions disponibles sont :
- Les mesures et régulations de température, pH, oxygène dissous, pression,
- La détection de mousse avec ajout régulé d'antimousse,
- Le contrôle de la procédure de stérilisation par vapeur vive,
- Le contrôle des pompes d'alimentation,
- La lecture des masses des balances.

La régulation pH est assurée deux pompes péristaltiques, l'une apportant une solution basique (hydroxyde potassium ou ammoniaque 10 mol.l⁻¹), l'autre apportant une solution acide (acide ortho phosphorique 0,29 mol.l⁻¹). Une troisième pompe péristaltique permet l'adjonction d'un anti-mousse (Strucktol) par ajout régulé.

Le bioréacteur est alimenté en substrat carboné (solution de glucose : 700 à 750 g/l) par commande de deux pompes volumétriques (fort et faible débit). Une troisième pompe, qui est asservie à l'apport de substrat, permet l'ajout d'une solution saline concentrée à un débit 10 fois inférieur à celui du substrat.

**Tableau 6 : composition de la solution saline d'alimentation pour Y. lipolytica**

| Composé | Concentration [g.l⁻¹] |
|---|---|
| KCl | 20,000 |
| NaCl | 20,000 |
| MgSO4;·7H₂O | 27,000 |
| ZnSO₄·; 7H₂O | 7,710 |
| MnSO₄·; H₂O | 0,470 |
| CoCl₂·; 6H₂O | 0,300 |
| CuSO₄·; 5H₂O | 0,600 |
| Na₂MoSO₄·; 2H₂O | 0,094 |
| CaCl₂·; 2H₂O | 6,400 |
| FeSO₄·; 7H₂O | 3,970 |
| H₃BO₃ | 0,300 |
| H₃PO₄ | 125,000 |

Les apports conjoints du milieu salin et du substrat carboné permettent d'assurer les besoins nutritionnelles de la biomasse (phase de croissance) hors azote. Une dernière pompe apporte l'azote de façon contrôlé à de très faibles débits lors de la phase d'accumulation de lipides sous limitation d'apport en azote.

Le débit massique de gaz entrant (oxygène apporté) est mesuré et régulé ainsi que la vitesse d'agitation (turbine Rhuston).

Le levain ensemençant la culture en réacteur est obtenu après une série de trois précultures :
1. préculture I de 8 ml de YPD pendant 24 h à 30 °C dans une fiole de 100 ml.
2. la préculture II de 72 ml de milieu minimum est ensemencée par la culture I, incubée pendant 12 h à 30 °C dans une fiole de 250 ml.
3. la préculture III de 720 ml de milieu minimum est ensemencée par la culture II, incubée pendant 12 h à 30 °C dans une fiole de 5000 ml. Le bioréacteur est alors ensemencé par cette dernière préculture.

Le milieu minimum est complémenté avec 0,8 g de glucose et 1 ‰ de solution concentrée de vitamines. La composition du milieu minimum et de la solution concentrée de vitamines sont décrites dans le tableau 7 et le tableau 8.

**Tableau 7 : composition du milieu minimum**

| Composé | Concentration [g.l⁻¹] |
|---|---|
| MgSO4·; 7H₂O | 5,278 |
| CaCl₂·; 2H₂O | 0,051 |
| FeSO₄·; 7H₂O | 0,215 |
| ZnSO₄·; 7H₂O | 0,078 |
| CuSO₄·; 5H₂O | 0,009 |
| CoCl₂·; 6H₂O | 0,158 |
| MnSO₄·; H₂O | 0,009 |
| Na₂MoO₄·2H₂O | 0,0360 |
| H₃PO₄ | 28,620 |
| H₂SO₄ | 16,370 |
| H₃BO₃ | 0,0025 |
| KCI | 7,450 |

**Tableau 8 : composition de la solution de vitamines 1000 fois concentrée**

| Composé | Concentration [g.l⁻¹] |
|---|---|
| d-biotine | 0,05 |
| Panthoténate | 1,00 |
| Acide nicotinique | 1,00 |
| Thiamine hydrocloride | 1,00 |
| Acide p-amino-benzoïque | 0,20 |
| Pyridoxol hydrocloride | 1,00 |
| Myoinositol | 25,00 |

Des échantillons sont régulièrement prélevés dans le réacteur, échantillons sur lesquels on réalise des mesures de :
- quantité de matière sèche correspondant à la biomasse totale selon la méthode gravimétrique après filtration d'un volume précis de suspension levurienne.
- quantité de lipides. la quantité de lipides totaux est déterminée selon une méthode dérivée de la méthode de Folch Folch J, Lees M, Slane-Stanley J (1957) A simple method for the isolation and purification of total lipids from animal tissues. J Biol Chem 226:497-509.

Trois cultures cellulaires ont été réalisées en environnement parfaitement contrôlé avec, en particulier, les pilotages du taux de croissance et de la cinétique d'accumulation des lipides par un contrôle minutieux de la limitation en azote. La première culture cellulaire de la souche sauvage *Y. lipolytica* W29 sert de culture référence afin de comparer les performances des deux souches mutantes Δgut2 et Δgut2Δpox1-6.

Les cultures cellulaires intègrent différentes phases qui sont :
- Phase 1 (t < 13-15 h) : croissance cellulaire en phase exponentielle (taux de croissance contrôlé par l'apport en substrat carboné).
- Phase 2 (13-15 h < t < 16-21 h) : mise en place de la limitation azote (basculement d'une régulation du pH de l'ammoniaque à l'hydroxyde de potassium), début d'accumulation des lipides (apport du substrat carboné en excès) et ralentissement de la croissance cellulaire (contrôle du taux de croissance par un apport d'azote externe).
- Phase 3 (t > 21 h) : accumulation

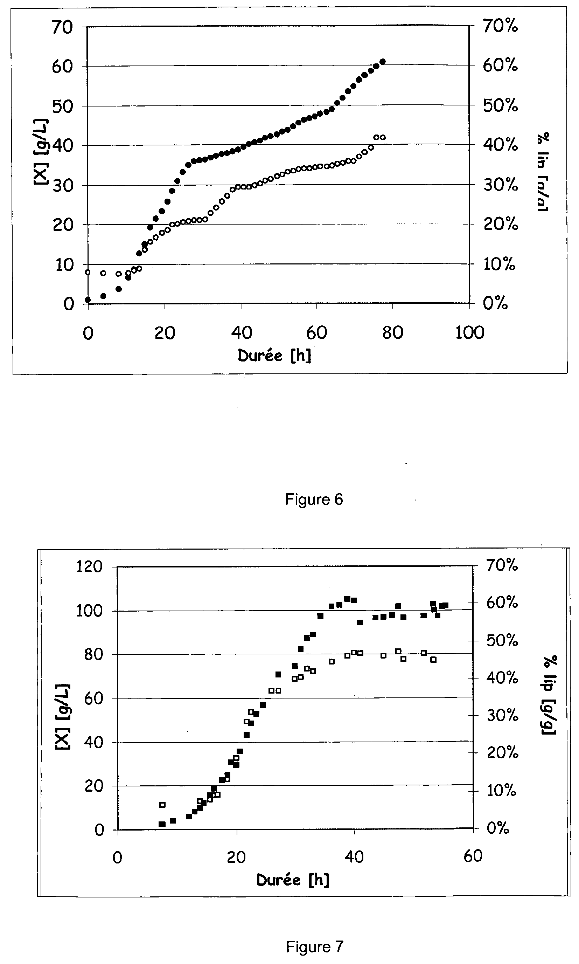

Les résultats de la culture en réacteur de la souche sauvage W29 (Figure 6) montrent une concentration cellulaire maximale de 60 g.l⁻¹ et une teneur maximale de lipides de 0.42 gₗᵢₚ.gₓ⁻¹ après 78 h de culture en réacteur. Lors de cette culture la phase d'accumulation de lipide a été initiée à 16 h. Il n'y a aucune phase de consommation de lipides intracellulaires.

Les résultats de la culture en réacteur de la souche mutante Δgut2 (Figure 7) montrent une concentration cellulaire maximale de 103 g.l⁻¹ soit une augmentation de plus de 70 % par rapport à la souche sauvage et une teneur maximale de lipides de 0.47 gₗᵢₚ.gₓ⁻¹ après 47 h soit une augmentation de 13 % de culture en réacteur en un temps 40 % plus court. Lors de cette culture la phase d'accumulation de lipide a été initiée à 18 h. Il n'y a aucune phase de consommation de lipides intracellulaires mais la teneur en lipides atteint une asymptote horizontale tendant vers 0.47 gₗᵢₚ.gₓ⁻¹.

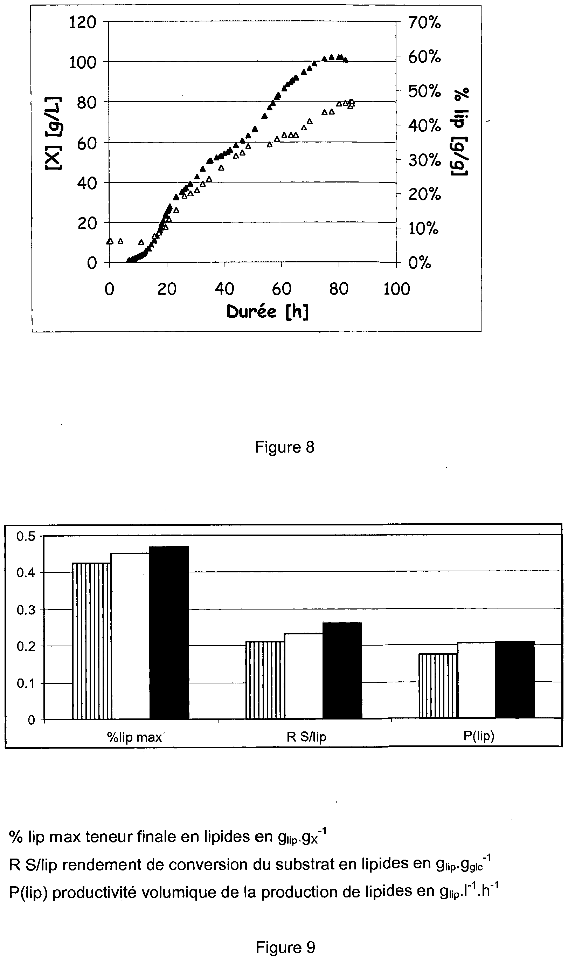

Les résultats de la culture en réacteur de la souche mutante Δgut2Δpox1-6 (Figure 8) montrent une concentration cellulaire maximale de 102 g.l⁻¹ soit une augmentation de plus de 70 % par rapport à la souche sauvage et une teneur maximale de lipides de 0.47 gₗᵢₚ.gₓ⁻¹ après 84 h soit une augmentation de 13% de culture en réacteur en un temps 40 % plus court. Lors de cette culture la phase d'accumulation de lipide a été initiée à 21 h. Il n'y a aucune phase de consommation de lipides intracellulaires mais la culture a été stoppée au début de la stagnation de la teneur en lipides.

### Bilan (Figure 9)

En suivant les rendements et productivités instantanées, il est possible de comparer les souches à l'aide des meilleures performances obtenues lors de chaque culture.

On remarque que la souche Δgut2 présente un rendement de conversion du glucose en lipides totaux supérieur de 10 % par rapport à la souche sauvage et d'une productivité volumétrique ([gₗᵢₚ-l⁻¹.h⁻¹]) supérieure de 19 %. En ce qui concerne la souche Δgut2Δpox1-6, l'amélioration des performances par rapport à la souche sauvage est de 24 % au niveau du rendement et de 20 % au niveau de la productivité.

## Revendications

1. Souche de levure *Yarrowia lipolytica,* mutante, n'exprimant pas le gène GUT2, ladite souche mutante étant capable d'accumuler des lipides, **caractérisée en ce qu'**elle n'exprime pas au moins un gène responsable de la β-oxydation des lipides, choisi parmi les gènes POX (POX1 à POX6), le gène MFE1 ou le gène POT1.

2. Souche de levure selon la revendication 1, **caractérisée en ce qu'**elle n'exprime pas les gènes POX2 à POX5.

3. Souche de levure selon la revendication 2, **caractérisée en ce qu'**elle n'exprime pas les gènes POX1 à POX6.

4. Souche de levure selon la revendication 3, **caractérisée en ce que** ladite souche est la souche |delta|gut2|delta|pox1-6. déposée auprès de la Collection Nationale de Cultures de Microorganismes (CNCM, Institut Pasteur, 25, rue du Docteur Roux, F-75724 Paris Cedex 15) sous le n°CNCM I-4169, le 28 mai 2009.

5. Procédé d'obtention d'une souche de levure *Yarrowia lipolytica,* mutante, n'exprimant pas le gène GUT2, capable d'accumuler des lipides, **caractérisé en ce que** :
5.1 dans une première étape, on construit une cassette d'invalidation comprenant les séquences promotrices (P) et terminatrices (T) du gène GUT2 de levure, encadrant un gène codant un marqueur de sélection (gène de sélection), ledit gène de sélection étant lui-même encadré de part et d'autre de sa séquence par une (ou des) séquence(s) de recombinaison;
5.2 dans une deuxième étape, on introduit ladite cassette d'invalidation obtenue à la première étape, dans une souche de levure ;
5.3 dans une troisième étape, on sélectionne, parmi les souches de levure transformées à la seconde étape, une souche de levure, défective en gène GUT2;
5.4 dans une quatrième étape, on vérifie l'invalidation dudit gène GUT2 dans ladite souche de levure sélectionnée à la troisième étape.

6. Procédé selon la revendication 5, **caractérisé en ce que** dans une cinquième étape (5.5) on transforme ladite souche sélectionnée à la quatrième étape avec un vecteur permettant l'expression d'une recombinase et dans une sixième étape (5.6), on isole une souche de levure défective en gène GUT2, et n'exprimant plus le gène marqueur.

7. Procédé selon la revendication 5, **caractérisé en ce que** à la première étape, on construit ladite cassette d'invalidation comprenant les séquences promotrices et terminatrices du gène GUT2 de levure, selon une méthode dans laquelle
7.1 dans un premier temps on synthétise et on purifie des fragments d'ADN comprenant soit la séquence Promotrice (P) soit la séquence Terminatrice (T) du gène GUT2 de levure, à partir d'ADN par exemple de l'ADN génomique de levure, avantageusement une souche sauvage, encore plus avantageusement une souche W29, préalablement purifié ;
7.2 dans un deuxième temps on solidarise un fragment P et un fragment T obtenus précédemment par l'intermédiaire du site de restriction, préférentiellement un site rare, avantageusement le site I-Scel, pour former des fragments d'ADN P-site rare-T (appelés cassettes d'invalidation 2) ;
7.3 dans un troisième temps on introduit au site de restriction de la cassette d'invalidation 2, un gène codant un marqueur de sélection, comme par exemple un gène associé à un phénotype d'auxotrophie ou de caractère dominant, pour former des fragments d'ADN P-I-Sce I-Marqueur-I-Sce I-T (cassette d'invalidation 1) et on purifie ladite cassette d'invalidation 1.

8. Procédé selon la revendication 7, **caractérisé en ce que** dans le premier temps on synthétise les fragments d'ADN comprenant soit la séquence Promotrice (P) soit la séquence Terminatrice (T) du gène GUT2 de levure, par PCR.

9. Procédé selon la revendication 8, **caractérisé en ce que** la PCR est réalisée sur de l'ADN de *Yarrowia lipolytica* en utilisant comme amorce les paires G3P-P1/ G3P-P2 (SEQ ID N°2 et 3) pour la partie P et G3P-T1/ G3P-T2 (SEQ ID N°4 et 5) pour la partie T.

10. Procédé selon la revendication 9, **caractérisé en ce que** les amorces G3P-P2 et G3P-T1 comportent en outre chacune une séquence d'un site de restriction, préférentiellement un site rare, comme par exemple un site choisi parmi les méganucléases, préférentiellement le site I-Scel.

11. Procédé selon la revendication 5, **caractérisé en ce qu'**à la seconde étape (5.2) on introduit la cassette d'invalidation 1 dans une souche de levure sauvage comme par exemple une souche W29 ou avantageusement une souche de levure n'exprimant pas le gène de marqueur de sélection contenu dans la cassette d'invalidation.

12. Procédé selon la revendication 11, **caractérisé en ce que** la souche de levure, n'exprimant pas le gène de marqueur de sélection contenu dans la cassette d'invalidation est une souche Po1d.

13. Procédé selon la revendication 5, **caractérisé en ce que** la souche de levure, mutante est la souche |delta|gut2 déposée auprès de la Collection Nationale de Cultures de Microorganismes (CNCM, Institut Pasteur, 25, rue du Docteur Roux, F-75724 Paris Cedex 15) sous le n°CNCM I-4038, le 8 Juillet 2008.

14. Procédé selon la revendication 5, **caractérisé en ce que** la souche de levure mutante est une souche qui n'exprime pas en outre au moins un gène responsable de la β-oxydation des lipides, choisi parmi les gènes POX (POX1 à POX6), le gène MFE1 ou le gène POT1.

15. Procédé selon la revendication 14, **caractérisé en ce que** la souche de levure n'exprime pas les gènes POX2 à POX5.

16. Procédé selon la revendication 15, **caractérisé en ce que** la souche de levure n'exprime pas les gènes POX1 à POX6.

17. Procédé selon la revendication 16, **caractérisé en ce que** la souche de Yarrowia mutante est la souche |delta|gut2|delta|pox1-6, déposée auprès de la Collection Nationale de Cultures de Microorganismes (CNCM, Institut Pasteur, 25, rue du Docteur Roux, F-75724 Paris Cedex 15) sous le n°CNCM I-4169. le 28 mai 2009.

18. Procédé selon la revendication14, **caractérisé en ce que** la souche de levure est choisie parmi les souches *MTLY37 (Leu+, Ura*+*, Δpox5, Δpox2, Δpox3, Δpox4 ::URA3), MTLY40 (Leu*+*, Ura*-, *Δpox5-PT, ΔpoX2-PT, Δpox3-PT, Δpox4::ura3-41), MTLY64 (Leu, Ura*-, *Hyg*+, *Δpox5, Δpox2, Δpox3, Δpox4::ura3-41, leu2 ::Hyg), MTLY66 (Leu-, Ura-, Δpox5, Δpox2, Δpox3, Δpox4::ura3-41, Δleu2), MTLY82 (Leu*-, *Ura*-, *Hyg*+, *Δpox5, Δpox2, Δpox3, Δpox4::ura3-41, Δleu2, pox1::Hyg), MTLY85 (Leu*-, *Ura*-, *Δpox5, Δpox2, Δpox3, Δpox4::ura3-41, Δleu2, Δpox1), MTLY92 (Leu*-, *Ura*-, *Hyg*+, *Δpox5, Δpox2, Δpox3, Δpox4::ura3-41, Δletu2, Δpox1, pox6::Hyg), et MTLY95a (Leu*-, *Ura*-, *Δpox5, Δpox2, Δpox3, Δpox4::ura3-41, Δleu2, Δpox1, Δpox6).*

19. Utilisation d'une souche de levure de *Yarrowia lipolytica,* mutante, n'exprimant pas le gène GUT2, pour la synthèse de lipides.

20. Utilisation selon la revendication 19, **caractérisée en ce que** la souche de *Yarrowia lipolytica,* mutante, est la souche |delta|gut2 déposée auprès de la Collection Nationale de Cultures de Microorganismes (CNCM, Institut Pasteur, 25, rue du Docteur Roux, F-75724 Paris Cedex 15) sous le n°CNCM I-4038, le 8 juillet 2008.

21. Utilisation selon la revendication 19, **caractérisée en ce que** la souche de Yarrowia mutante est la souche|delta|gut2|delta|pox1-6, déposée auprès de la Collection Nationale de Cultures de Microorganismes (CNCM, Institut Pasteur, 25, rue du Docteur Roux, F-75724 Paris Cedex 15) sous le n°CNCM I-4169, le 28 mai 2009.

22. Procédé de synthèse de lipides dans lequel
22.1 dans une première étape on cultive une souche de levure *Yarrowia lipolytica,* mutante, n'exprimant pas le gène GUT2, capable d'accumuler des lipides, dans un milieu approprié ;
22.2 dans une seconde étape on récolte les lipides produits par la culture de l'étape 1.

23. Utilisation selon la revendication 19, pour la synthèse des acides gras libres et des triacylglycérols.

24. Utilisation selon la revendication 19, pour la production d'acides gras n-2.

25. Utilisation selon la revendication 19, pour la production du 16:1 (n-9), l'acide (Z)-7-hexadedecenoique.

## Patentansprüche

1. Mutanter Hefestamm *Yarrowia lipolytica,* der das Gen GUT2 nicht exprimiert, wobei der genannte mutante Stamm Lipide akkumulieren kann, **dadurch gekennzeichnet, dass** er wenigstens ein Gen nicht exprimiert, das für die β-Oxydation von Lipiden verantwortlich ist, ausgewählt aus den Genen POX (POX1 bis POX6), MFE1 und POT1.

2. Hefestamm nach Anspruch 1, **dadurch gekennzeichnet, dass** er die Gene POX2 bis POX5 nicht exprimiert.

3. Hefestamm nach Anspruch 2, **dadurch gekennzeichnet, dass** er die Gene POX1 bis POX6 nicht exprimiert.

4. Hefestamm nach Anspruch 3, **dadurch gekennzeichnet, dass** der genannte Stamm der Stamm |delta|gut2|delta|pox1-6 ist, hinterlegt bei der Collection Nationale de Cultures de Microorganismes (CNCM, Institut Pasteur, 25, rue du Docteur Roux, F-75724 Paris Cedex 15) unter der Nr. CNCM 1-4169, am 28. Mai 2009.

5. Verfahren zur Gewinnung eines mutanten Hefestamms *Yarrowia lipolytica,* der das Gen GUT2 nicht exprimiert, der Lipide akkumulieren kann, **dadurch gekennzeichnet, dass**:
5.1 in einem ersten Schritt eine Ausschaltungskassette konstruiert wird, die die Promotor- (P) und Terminatorsequenzen (T) des Hefegens GUT2 umfasst, das ein Gen flankiert, das einen Selektionsmarker (Selektionsgen) kodiert, wobei das genannte Selektionsgen wiederum auf beiden Seiten seiner Sequenz von einer oder mehreren Rekombinationssequenzen flankiert wird;
5.2 in einem zweiten Schritt die im ersten Schritt gewonnene Ausschaltungskassette in einen Hefestamm eingeführt wird;
5.3 in einem dritten Schritt ein im Gen GUT2 defekter Hefestamm aus den im zweiten Schritt transformierten Hefestämmen ausgewählt wird;
5.4 in einem vierten Schritt die Ausschaltung des genannten Gens GUT2 in dem genannten im dritten Schritt ausgewählten Hefestamm überprüft wird.

6. Verfahren nach Anspruch 5, **dadurch gekennzeichnet, dass** in einem fünften Schritt (5.5) der genannte, im vierten Schritt gewählte Stamm mit einem Vektor transformiert wird, der die Expression einer Rekombinase zulässt, und in einem sechsten Schritt (5.6) ein im Gen GUT2 defekter Hefestamm isoliert wird, der das Markergen nicht mehr exprimiert.

7. Verfahren nach Anspruch 5, **dadurch gekennzeichnet, dass** im ersten Schritt die Ausschaltungskassette konstruiert wird, die die Promotor- und Terminatorsequenzen des Hefegens GUT2 umfasst, mit einer Methode, bei der:
7.1 zu einem ersten Zeitpunkt die DNA-Fragmente synthetisiert und gereinigt werden, die entweder die Promotorsequenz (P) oder die Terminatorsequenz (T) des Hefegens GUT2 umfassen, auf der Basis von DNA, zum Beispiel von genomischer Hefe-DNA, vorteilhafterweise von einem Wildstamm, stärker bevorzugt einem zuvor gereinigten Stamm W29;
7.2 zu einem zweiten Zeitpunkt ein P-Fragment und ein T-Fragment solidarisiert werden, die zuvor mittels der Restriktionsstelle, vorzugsweise einer seltenen Stelle, vorzugsweise der Stelle I-Scel erhalten wurden, zum Bilden der DNA-Fragmente P-Seltenstelle-T (Ausschaltungskassetten 2 genannt);
7.3 zu einem dritten Zeitpunkt an der Restriktionsstelle der Ausschaltungskassette 2 ein Gen eingeführt wird, das einen Selektionsmarker kodiert, wie zum Beispiel ein Gen in Verbindung mit einem Auxotrophie-Phänotyp oder mit dominantem Charakter, zum Bilden von DNA-Fragmenten P-I-Sce I-Marker-I-Sce I-T (Ausschaltungskassette 1), und die genannte Ausschaltungskassette 1 gereinigt wird.

8. Verfahren nach Anspruch 7, **dadurch gekennzeichnet, dass** zu einem ersten Zeitpunkt die DNA-Fragmente, die entweder die Promotorsequenz (P) oder die Terminatorsequenz (T) des Hefegens GUT2 enthalten, durch PCR synthetisiert werden.

9. Verfahren nach Anspruch 8, **dadurch gekennzeichnet, dass** die PCR auf der DNA von *Yarrowia lipolytica* unter Verwendung der Paare G3P-P1/ G3P-P2 (SEQ ID Nr. 2 und 3) als Ausgangspunkt für den Teil P und G3P-T1/ G3P-T2 (SEQ ID Nr. 4 und 5) für den Teil T benutzt wird.

10. Verfahren nach Anspruch 9, **dadurch gekennzeichnet, dass** die Ausgangspunkte G3P-P2 und G3P-T1 darüber hinaus jeweils eine Restriktionsstellensequenz umfassen, vorzugsweise eine seltene Stelle, wie zum Beispiel eine Stelle, die aus Meganukleasen ausgewählt ist, vorzugsweise die Stelle I-Scel.

11. Verfahren nach Anspruch 5, **dadurch gekennzeichnet, dass** im zweiten Schritt (5.2) die Ausschaltungskassette 1 in einen Wildhefestamm eingeführt wird, wie z.B. einen Stamm W29 oder vorteilhafterweise einen Hefestamm, der das in der Ausschaltungskassette enthaltene Selektionsmarkergen nicht exprimiert.

12. Verfahren nach Anspruch 11, **dadurch gekennzeichnet, dass** der Hefestamm, der das in der Ausschaltungskassette enthaltene Selektionsmarkergen nicht exprimiert, ein Stamm Po1d ist.

13. Verfahren nach Anspruch 5, **dadurch gekennzeichnet, dass** der mutante Hefestamm der Stamm |delta|gut2 ist, hinterlegt bei der Collection Nationale de Cultures de Microorganismes (CNCM, Institut Pasteur, 25, rue du Docteur Roux, F-75724 Paris Cedex 15) unter der Nr. CNCM 1-4038, am 8. Juli 2008.

14. Verfahren nach Anspruch 5, **dadurch gekennzeichnet, dass** der mutante Hefestamm ein Stamm ist, der darüber hinaus wenigstens ein Gen nicht exprimiert, das für die β-Oxydation von Lipiden verantwortlich ist, ausgewählt aus den Genen POX (POX1 bis POX6), dem Gen MFE1 und dem Gen POT1.

15. Verfahren nach Anspruch 14, **dadurch gekennzeichnet, dass** der Hefestamm die Gene POX2 bis POX5 nicht exprimiert.

16. Verfahren nach Anspruch 15, **dadurch gekennzeichnet, dass** der Hefestamm die Gene POX1 bis POX6 nicht exprimiert.

17. Verfahren nach Anspruch 16, **dadurch gekennzeichnet, dass** der mutante Yarrowia-Stamm der Stamm |delta|gut2|delta|pox1-6 ist, hinterlegt bei der Collection Nationale de Cultures de Microorganismes (CNCM, Institut Pasteur, 25, rue du Docteur Roux, F-75724 Paris Cedex 15) unter der Nr. CNCM 1-4169, am 28. Mai 2009.

18. Verfahren nach Anspruch 14, **dadurch gekennzeichnet, dass** der Hefestamm ausgewählt wird aus den Stämmen *MTLY37 (Leu+, Ura*+, *Δpox5, Δpox2, Δpox3, Δpox4::URA3), MTLY40 (Leu*+, *Ura*-, *Δpox5-PT, Δpox2-PT, Δpox3-PT, Δpox4::ura3-41), MTLY64 (Leu, Ura*-, *Hyg*+, *Δpox5, Δpox2, Δpox3, Δpox4::ura3-41, leu2::Hyg)*, *MTLY66 (Leu-, Ura*-, *Δpox5, Δpox2, Δpox3, Δpox4::ura3-41, Δleu2), MTLY82 (Leu*-, *Ura*-, *Hyg*+, *Δpox5, Δpox2, Δpox3, Δpox4::ura3-41, Δleu2, pox1::Hyg), MTLY85 (Leu-, Ura*-, *Δpox5, Δpox2, Δpox3, Δpox4::ura3-41, Δleu2, Δpox1), MTLY92 (Leu-, Ura-, Hyg+, Δpox5, Δpox2, Δpox3, Δpox4::ura3-41, Δleu2, Δpox1, pox6::Hyg), und MTLY95a (Leu-, Ura-, Δpox5, Δpox2, Δpox3, Δpox4::ure3-41, Δleu2, Δpox1, Δpox6).*

19. Verwendung eines mutanten Hefestamms *Yarrowia lipolytica,* der das Gen GUT2 nicht exprimiert, für die Synthese von Lipiden.

20. Verwendung nach Anspruch 19, **dadurch gekennzeichnet, dass** der mutante Stamm *Yarrowia lipolytica* der Stamm |delta|gut2 ist, hinterlegt bei der Collection Nationale de Cultures de Microorganismes (CNCM, Institut Pasteur, 25, rue du Docteur Roux, F-75724 Paris Cedex 15) unter der Nr. CNCM 1-4038, am 8. Juli 2008.

21. Verwendung nach Anspruch 19, **dadurch gekennzeichnet, dass** der mutante Stamm Yarrowia der Stamm |delta|gut2|delta|pox1-6 ist, hinterlegt bei der Collection Nationale de Cultures de Microorganismes (CNCM, Institut Pasteur, 25, rue du Docteur Roux, F-75724 Paris Cedex 15) unter der Nr. CNCM 1-4169, am 28. Mai 2009.

22. Verfahren zum Synthetisieren von Lipiden, bei dem
22.1 in einem ersten Schritt ein mutanter Hefestamm *Yarrowia lipolytica* kultiviert wird, der das Gen GUT2 nicht exprimiert, der Lipide akkumulieren kann, in einem geeigneten Medium;
22.2 in einem zweiten Schritt die durch die Kultur in Schritt 1 produzierten Lipide geerntet werden.

23. Verwendung nach Anspruch 19 für die Synthese von freien Fettsäuren und von Triacylglycerolen.

24. Verwendung nach Anspruch 19 für die Produktion von Fettsäuren n-2.

25. Verwendung nach Anspruch 19 für die Produktion von 16:1 (n-9), (Z)-7-Hexadecanoiesäure.

## Claims

1. Mutant *Yarrowia lipolytica* yeast strain, not expressing the GUT2 gene, said mutant strain being capable of accumulating lipids, **characterized in that** it does not express at least one gene responsible for the β-oxidation of lipids, chosen from the POX genes (POX1 to POX6), the MFE1 gene or the POT1 gene.

2. Yeast strain according to claim 1, **characterized in that** it does not express the POX2 to POX5 genes.

3. Yeast strain according to claim 2, **characterized in that** it does not express the POX1 to POX6 genes.

4. Yeast strain according to claim 3, **characterized in that** said strain is the |delta|gut2|delta|pox1-6 strain deposited with the Collection Nationale de Cultures de Microorganismes (CNCM, Institut Pasteur, 25, rue du Docteur Roux, F-75724 Paris Cedex 15) under No. CNCM 1-4169, on 28 May 2009.

5. Method for obtaining a mutant *Yarrowia lipolytica* yeast strain not expressing the GUT2 gene, capable of accumulating lipids, **characterized in that**:
5.1 in a first step, an invalidation cassette is constructed comprising the promoter (P) and terminator (T) sequences of the GUT2 yeast gene, flanking a gene coding for a selection marker (selection gene), said selection gene being itself flanked at each end of its sequence by one or more recombination sequence(s);
5.2 in a second step, said invalidation cassette obtained in the first step is introduced into a yeast strain;
5.3 in a third step, a yeast strain defective in GUT2 gene is selected from the yeast strains transformed in the second step;
5.4 in a fourth step, the invalidation of said GUT2 gene in said yeast strain selected in the third step is verified.

6. Method according to claim 5, **characterized in that** in a fifth step (5.5), said strain selected in the fourth step is transformed with a vector allowing the expression of a recombinase and in a sixth step (5.6), a yeast strain is isolated that is defective in GUT2 gene, no longer expressing the marker gene.

7. Method according to claim 5, **characterized in that** in the first step, said invalidation cassette is constructed, comprising the promoter and terminator sequences of the GUT2 yeast gene, according to a method in which
7.1 firstly, DNA fragments are synthesized and purified comprising either the Promoter sequence (P) or the Terminator sequence (T) of the GUT2 yeast gene, starting from DNA, for example yeast genomic DNA, advantageously a wild-type strain, even more advantageously a W29 strain purified beforehand;
7.2 secondly, a fragment P and a fragment T, obtained previously via the restriction site, preferentially a rare site, advantageously the I-Scel site, are joined in order to form P-rare site-T DNA fragments (called invalidation cassettes 2);
7.3 thirdly, a gene coding for a selection marker, such as for example a gene associated with an auxotrophic phenotype or one of a dominant character, is introduced into the restriction site of the invalidation cassette 2 in order to form P-I-Sce I-Marker-I-Sce I-T DNA fragments (invalidation cassette 1) and said invalidation cassette 1 is purified.

8. Method according to claim 7, **characterized in that** firstly, the DNA fragments comprising either the Promoter sequence (P) or the Terminator sequence (T) of the GUT2 yeast gene are synthesized by PCR.

9. Method according to claim 8, **characterized in that** the PCR is performed on DNA of *Yarrowia lipolytica* using as primers the pairs G3P-P1/ G3P-P2 (SEQ ID No.2 and 3) for the part P and G3P-T1/ G3P-T2 (SEQ ID No.4 and 5) for the part T.

10. Method according to claim 9, **characterized in that** the G3P-P2 and G3P- T1 primers each also contain a restriction site sequence, preferentially a rare site, such as for example a site chosen from the meganucleases, preferentially the I-Scel site.

11. Method according to claim 5, **characterized in that** in the second step (5.2) the invalidation cassette 1 is introduced into a wild-type yeast strain such as for example a W29 strain or advantageously a yeast strain not expressing the selection marker gene contained in the invalidation cassette.

12. Method according to claim 11, **characterized in that** the yeast strain not expressing the selection marker gene contained in the invalidation cassette is a Po1d strain.

13. Method according to claim 5, **characterized in that** the mutant yeast strain is the |delta|gut2 strain deposited with the Collection Nationale de Cultures de Microorganismes (CNCM, Institut Pasteur, 25, rue du Docteur Roux, F-75724 Paris Cedex 15) under No. CNCM 1-4038, on 8 July 2008.

14. Method according to claim 5, **characterized in that** the mutant yeast strain is a strain which also does not express at least one gene responsible for the β-oxidation of lipids, chosen from the POX genes (POX1 to POX6), the MFE1 gene or the POT1 gene.

15. Method according to claim 14, **characterized in that** the yeast strain does not express the POX2 to POX5 genes.

16. Method according to claim 15, **characterized in that** the yeast strain does not express the POX1 to POX6 genes.

17. Method according to claim 16, **characterized in that** the mutant *Yarrowia* strain is the |delta|gut2|delta|pox1-6 strain, deposited with the Collection Nationale de Cultures de Microorganismes (CNCM, Institut Pasteur, 25, rue du Docteur Roux, F-75724 Paris Cedex 15) under No. CNCM 1-4169 on 28 May 2009.

18. Method according to claim 14, **characterized in that** the yeast strain is chosen from the strains *MTLY37 (Leu+, Ura+, Δpox5, Δpox2, Δpox3, Δpox4::URA3), MTLY40 (Leu+, Ura-, Δpox5-PT, Δpox2-PT, Δpox3-PT, Δpox4::ura3-41), MTLY64 (Leu, Ura-, Hyg+, Δpox5, Δpox2, Δpox3, Δpox4::ura3-41, leu2::Hyg), MTL Y66 (Leu-, Ura-, Δpox5, Δpox2, Δpox3, Δpox4::ura3-41, Δleu2), MTLY82 (Leu-, Ura-, Hyg+, Δpox5, Δpox2, Δpox3, Δpox4::ura3-41, Δleu2, pox1::Hyg), MTLY85 (Leu-, Ura-, Δpox5, Δpox2, Δpox3, Δpox4::ura3-41, Δleu2, Δpox1), MTLY92 (Leu-, Ura-, Hyg+, Δpox5, Δpox2, Δpox3, Δpox4::ura3-41, Δleu2, Δpox1, pox6::Hyg), and MTLY95a (Leu-, Ura-, Δpox5, Δpox2, Δpox3, Δpox4::ure3-41, Δleu2, Δpox1, Δpox6).*

19. Use of a mutant *Yarrowia lipolytica* strain not expressing the GUT2 gene, for the synthesis of lipids.

20. Use according to claim 19, **characterized in that** the mutant *Yarrowia lipolytica* strain is the |delta|gut2 strain deposited with the Collection Nationale de Cultures de Microorganismes (CNCM, Institut Pasteur, 25, rue du Docteur Roux, F- 75724 Paris Cedex 15) under No. CNCM 1-4038, on 8 July 2008.

21. Use according to claim 19, **characterized in that** the mutant *Yarrowia* strain is the |delta|gut2|delta|pox1-6 strain, deposited with the Collection Nationale de Cultures de Microorganismes (CNCM, Institut Pasteur, 25, rue du Docteur Roux, F-75724 Paris Cedex 15) under No. CNCM 1-4169, on 28 May 2009.

22. Method for the synthesis of lipids in which
22.1 in a first step, a mutant *Yarrowia lipolytica* yeast strain not expressing the GUT2 gene, capable of accumulating lipids, is cultured in a suitable medium;
22.2 in a second step, the lipids produced by the culture of step 1 are harvested.

23. Use according to claim 19, for the synthesis of free fatty acids and triacylglycerols.

24. Use according to claim 19, for the production of n-2 fatty acids.

25. Use according to claim 19, for the production of 16:1 (n-9), (Z)-7-hexadecenoic acid.
